# EUROPEAN PATENT APPLICATION

(11) **EP 2 033 512 A1**
(43) Date of publication of application: **11.03.2009**
(21) Application number: 07766954.7
(22) Date of filing: 26.06.2007
(51) Int. Cl.: A01K 67/027, A61K 38/00, A61P 31/04, A61P 31/10, A61P 31/12, A61P 33/02, A61P 35/00, A61P 37/04, C12N 15/09, C12Q 1/02, C12Q 1/68, G01N 33/15, G01N 33/50, G01N 33/53, C07K 14/705

(54) **MODEL ANIMAL OF DENDRITIC CELL IMMUNORECEPTOR GENE KNOCKOUT DISEASE**

(30) Priority: 28.06.2006 JP 2006177952; 09.02.2007 JP 2007030514
(71) Applicant: Genodive Pharma Inc., Isehara-shi, Kanagawa 2591144 (JP); Iwakura, Yoichiro, Tokyo 150-0013 (JP)
(72) Inventor: IWAKURA, Yoichiro, Tokyo 150-0013 (JP); SAIJO, Shinobu, Tokyo 141-0032 (JP); FUJIKADO, Noriyuki, Tokyo 158-0085 (JP)
(74) Representative: Docherty, Robert Charles
(86) International application number: PCT/JP2007/000696
(87) International publication number: WO 2008/001498

(57) **Abstract**

It is intended to disclose the function of dendritic cell immunoreceptor (DCIR) and clarify its role in the onset of autoimmune arthritis. It is also intended to provide a method of screening a substance which is useful in treating/preventing an autoimmune disease or osteoporosis. A nonhuman disease model animal **characterized by** having a partial or entire deficiency of a gene encoding the DCIR protein on the chromosome; a method of screening a substance which is useful in treating/preventing a DCIR-related disease, for example, an autoimmune disease such as arthropathy by using the nonhuman disease model animal as described above; and a remedy/preventive therefor.

## Description

### Field of the invention

The present invention relates to a model animal of autoimmune diseases such as arthropathy, and bone diseases, characterized by having deficiency of a dendritic cell immunoreceptor gene (knockout (KO)). Furthermore, the invention relates to a screening method of a preventive/remedy using the model animal, and diagnosis and/or treatment of autoimmune diseases and bone diseases provided by the screening method.

### Background Art

Rheumatoid arthritis (RA) is systemic chronic inflammatory disease mainly affecting synovial membrane at many joints. Inflammation of synovial membrane leads to damaged cartilage, bone erosion, deformed joint and loss of joint function. RA is an autoimmune disease characterized by infiltration of T-cells, B-cells, macrophage and neutrophil into surface of synovial membrane and fluid in cavities around joints.

The inventors have already established two types of model mice for RA by gene engineering of embryo. These are a human T cell leukemia virus type I transgenic (HTLV-I-Tg) mouse (patent document 1), and an IL-1 receptor antagonist defective (IL-1 Ra-/-) mouse (patent document 2), that spontaneously develop autoimmune and arthritis. The histopathological characteristics of the affected joints of these animals highly resemble that of human RA. The inventors conducted an exhaustive microarray analysis of gene expression on these two types of arthritis model mice, and found strong correlation in gene expression profile between a HTLV-I Tg mouse and an IL-1 Ra-/- mouse. In spite of apparent etiological difference between these mice, they showed similarity not only in histology but also in gene expression profile.

Particularly, the inventors newly found significant increase in expression of some genes on chromosome 6F2 site band of these RA model mice. The genes include calcium dependent (c type) lectin superfamily genes. C type lectine receptor (CLR) is a pattern recognition molecule that recognizes specific carbohydrate structure on the autoantigen or cell wall of pathogens, by extracellular carbohydrate recognition domain (CRD). CLRs including macrophage mannose receptor (MMR: CD206), dendritic cell specific ICAM3-grabbing non-integrin (DC-SIGN: CD209), L-SIGN and β-GR (Dectin-1) are-involved in recognition of various kinds of microbes such as virus, bacteria, fungus and some parasites. Interestingly, it is reported that signal transduction of CLRs are facilitated by that of Toll-like receptor (TLR), indicating possible cross-talk between these 2 signal transduction pathways. Also, CLRs regulate migration of dendritic cells and their interaction with lymphocytes. Other members of CLRs on NK cells such as human CD94/NKG2 and mouse Ly49 are involved in recognition of MHC class I to discern self from non-self. In short, CLRs play a role in natural immunity as well as acquired immunity. Some CLRs have signal transduction motifs such as immunoreceptor tyrosine-based inhibitory motif (ITIM) or immunoreceptor tyrosine-based activating motif (ITAM) in cytoplasm, which may regulate expression of function of dendritic cells.

Dendritic cells (DCs) are one of major antigen presentation cells, playing an essential role in regulation of immune system. Recently, expression of some CLRs on the surface of dendritic cells has been identified and characterization was made. MMR (CD206) and DEC-205 (CD205) belonging to type I CLR have plural calcium dependent extracellular carbohydrate recognition domains (CRDs) at its N-terminal. The second family of CLRs expressed on dendritic cells is type II protein having single CRD on its C-terminal, including DC-SIGN (CD209), Langerin (CD207), CLEC-1, Dectin-1 (β-GR), Dectin-2, DLEC and DCIR.

Among them, DCIR, that is also referred to as LLIR, is a type II membrane protein expressed mainly on dendritic cells of human and mouse. This molecule has single CRD in its extracellular domain, and a consensus ITIM domain in its intracellular domain. Since ITIM transmits inhibitory signal in the cell, it is suggested that mouse DCIR might serve as an inhibitory receptor to regulate function of dendritic cells. However, in vivo evidence of DCIR function has not been reported as of yet.

Patent Document 1: Japanese published unexamined application 6-38652
Patent Document 2: Japanese published unexamined application 2000-209980

### Summary of the invention

### Problems to be solved by the invention

The present invention was made on the inventors' findings about dendritic cell immunoreceptor (DCIR) of which expression is increased in the two types of mouse models established for rheumatoid arthritis (RA), one of autoimmune arthritis. The purposes of the invention are to provide a DCIR defective (KO) mouse (DCIR-/- mouse) to elucidate the role of DCIR in development of autoimmune diseases including arthritis, to demonstrate the usefulness of the DCIR-/- mouse as a disease model animal, to provide a method to screen a preventive/remedy for autoimmune diseases using the DCIR-/- mouse, and to provide a preventive/remedy for the autoimmune diseases using the screening method.

### Means to solve the problem

The objectives of the inventions are to:
(1) provide a nonhuman disease model animal characterized by having a partial or entire deficiency of a gene coding dendritic cell immunoreceptor (DCIR) protein on the chromosome; and
(2) provide a screening method of a substance that suppresses or facilitates differentiation and proliferation of bone-marrow stem cells derived cells, or a substance that suppresses development of arthritis, and to provide a preventive/remedy for autoimmune diseases containing compound or salt thereof identified by the screening method.

Further objectives of the invention are to:
(3) provide a preventive/remedy for autoimmune diseases comprising a substance that facilitates activity of DCIR protein or expression of gene coding DCIR protein, and to provide a diagnostic agent for autoimmune diseases comprising an entire or partial gene coding DCIR protein; and,
(4) provide a kit for screening a preventive/remedy for autoimmune diseases comprising a peptide having amino acid sequence identical or substantially identical to DCIR protein, a partial peptide or a salt thereof, and a diagnostic kit for autoimmune disease comprising a partial or entire DNA having base sequence identical or substantially identical to the gene coding DCIR protein.

### Effects of the invention

The DCIR defective mouse (KO mouse, hereinafter referred to as DCIR-/- mouse) according to the invention shows elevated sensitivity to collagen induced arthritis (CIA), thus, it is useful as a disease model animal of autoimmune diseases including arthritis. Also, an aged DCIR-/- mouse spontaneously develops autoimmune disease-like pathology such as inflammation at insertion of tendon or ligament to bone (enthesitis) or sialadenitis, which indicates regulation of immune system by DCIR. Thus, the disease model mouse according to the invention is markedly useful as an autoimmune diseases model mouse, the diseases including RA, ankylosing spondylitis (AS), diffused idiopathic skeletal hyperostosis (DISH), and Sjogren syndrome (SS).
Furthermore, the disease model animal according to the invention shows increased calcification at heel joint and decreased bone mass at femur compared to wild type animal. Thus, the mouse can be used as a model animal of bone diseases.
Also, the DCIR-/- mouse according to the invention shows increased anti-tumor immunity. Since DCIR plays a role in inhibitory function of dendritic cells, DCIR protein or equivalent thereof may be used as an activating agent in immunotherapy of cancer.
The model animal according to the invention allows analysis of physiological function and autoimmune disease inducing function of DCIR. Also, it can be used to elucidate mechanism of bone and cartilage diseases such as RA, AS, DISH or osteoporosis and to develop effective method and drug for treatment and diagnosis.

### Brief description of drawings

Fig 1a is a graph that shows expression of DCIR mRNA in joints of an IL-1Ra-/- mouse and a HTL V-T-Tg mouse with arthritis by microarray analysis.
Fig 1b shows expression of DCIR mRNA by northern blotting.
Fig. 2a is a schematic view that shows structures of DCIR locus on mouse DNA (wild type allele), DCIR targeting construct (targeting vector), and putative mutant DCIR gene (mutant allele), in which exons are indicated by boxes, that are used for production of a DCIR-/- mouse. Exon 1 and 2 of DCIR gene are substituted with neomycin resistance (Neo) gene. Diphtheria toxin (DT) gene is ligated to 3' terminal of genome fragment for negative selection. External homologous region in the targeting allele is used as a genome probe for southern blotting. Southern blotting for screening used BanHI.
Fig. 2b is a drawing that shows the result of southern blot hybridization using a genome DNA fragment cleaved by BanHI of targeted ES clone and 5' probe.
Fig. 2c is a drawing that shows the result of southern blot hybridization using a genome DNA fragment cleaved by EcoRI and 3' probe.
Fig. 2d is a drawing that shows the result of southern blot hybridization using a genome DNA fragment cleaved by EcoRI and Neo probe.
Fig. 2e is a drawing that shows a result of southern blot hybridization to ensure proper targeting of DCIR region on a mouse DNA.
Fig. 2f is a drawing that shows expression of DCIR mRNA by northern blotting.
Fig3 is a drawing that shows characteristics of lymphocyte in a DCIR-/- mouse. This is a result of flow cytometry of thymus cells and spleen cells of a wild type (WT) mouse and a DCIR-/- mouse. For thymus cells and spleen cells, expression of CD4 and CD8 was analyzed, and for spleen cells, expression of CD3 and B220 was analyzed as well.
Figs.4a and 4b are drawings that show proliferation of T-cells dependent on dendritic cells.

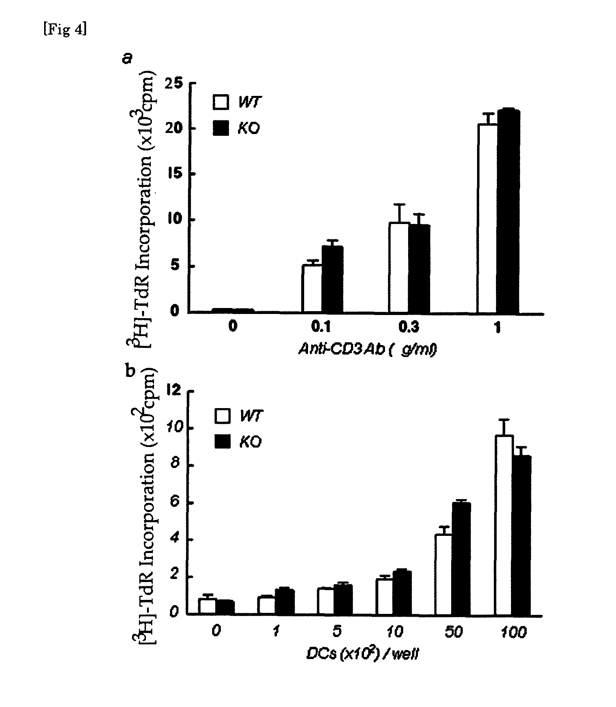

Fig. 4a is a graph that shows incorporation of tritium thymidine by T cells derived from a spleen of a WT mouse when the T cells are co-cultured with dendritic cells derived from a WT mouse (WT) or a DCIR-/- mouse (KO), under presence of antigen nonspecific stimulus (anti CD3 antibody).
Fig. 4b is a graph that shows incorporation of tritium thymidine by T cells derived from a BALB/cA mouse (H-2^{d}) when the T cells are co-cultured with dendritic cells derived from a WT mouse (WT) or a DCIR-/- mouse (KO) with 129/Sv x C57BU6) F1 background.

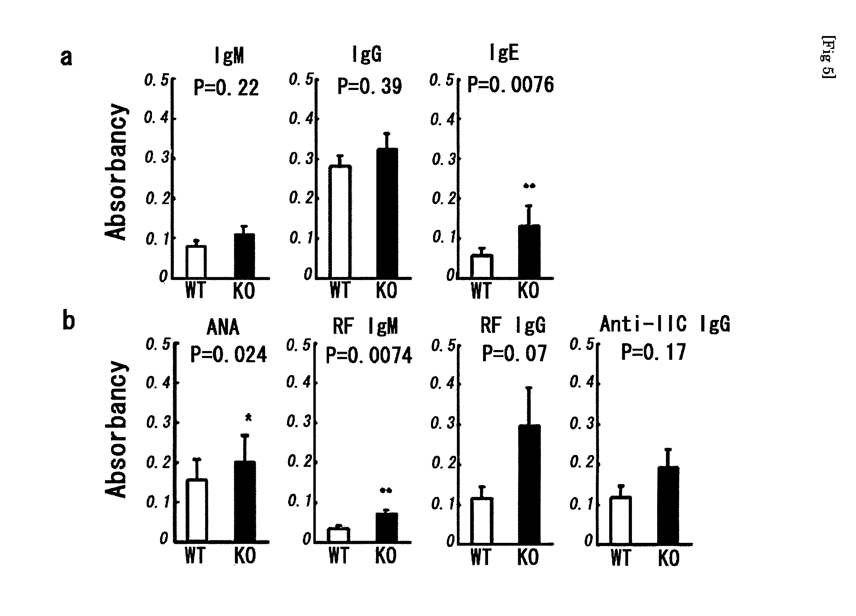

Figs. 5a and 5b are graphs that show elevated production of autoantibody in DCIR-/- mice. The serum antibody level of DCIR-/- mice (n=19) and WT mice litter (n=19) at 12 months of age was determined by ELISA.
Fig. 5a shows total IgM, IgG and IgE levels.

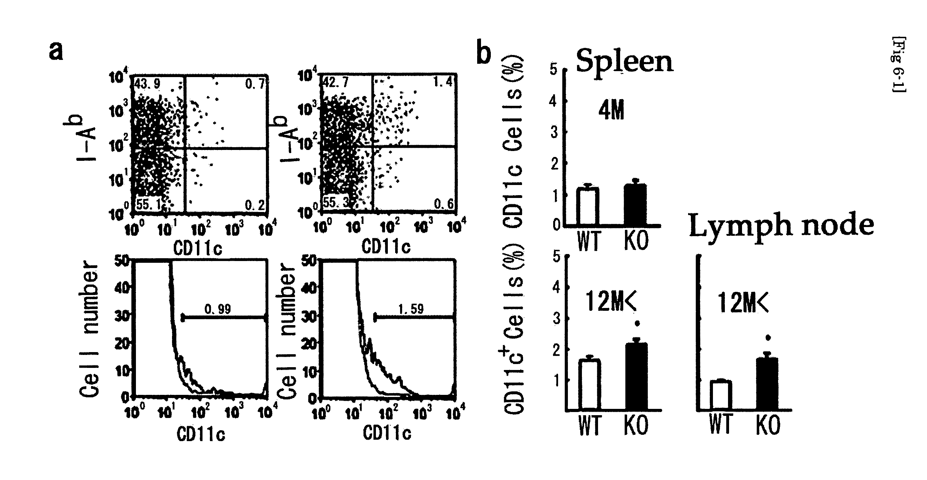

Fig. 5b shows autoimmune antibodies level, particularly, it shows levels of antinuclear antibody (ANA), rheumatoid factor (RF IgM and RF IgG) and anti-IIC antibody. The graph illustrates mean and SEM. *p<0.05, ** p<0.01 Figs. 6a to 6d are drawings that show accumulation of dendritic cells and activation of T cells in aged DCIR-/- mice.
Fig. 6a is a drawing that shows stained CD11c and I-A^{b} of lymph node cells. The figures in the drawing shows rate of cells in a specific gate while the histogram at the bottom shows CD11c positive clusters in lymph node cells.
Fig. 6b is a graph that shows the result of flow cytometry to determine rate of CD11c positive cells in the lymph node cells of mice at 4 and 12 or above months of age. The graph illustrates mean and SEM. * p<0.05.

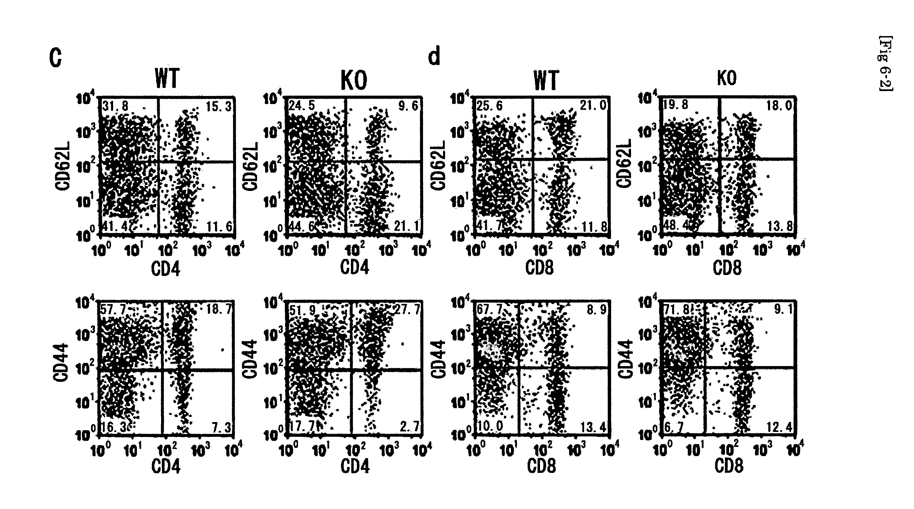

Fig. 6c is a drawing that shows stained CD4 and CD62L or CD44 on lymph node cells derived from a WT mouse and a DCIR-/- mouse at 12 months of age.
Fig. 6d shows a drawing that shows stained CD8 and CD62L or CD44.

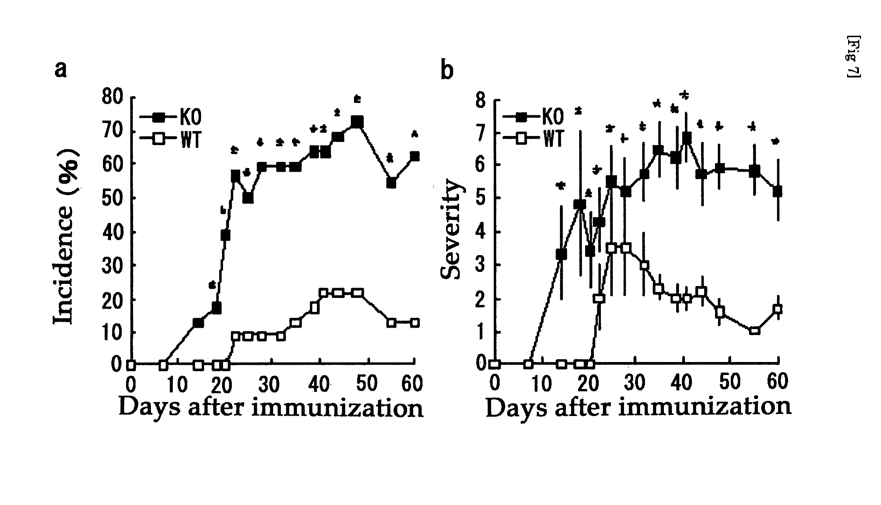

Figs. 7a and 7b are graphs that show aggravation of CIA in DCIR-/- mice. Chicken IIC was suspended with CFA, and injected at a plurality of percutaneous sites on the mouse tail. Development of CIA was observed without additional immunization. Fig. 7a shows incidence and 7b shows severity of CIA. The graphs illustrate mean and SEM of the pooled data of independent two tests. For statistical analysis, c² test is used for the test shown in Fig. 7a while Mann-Whitney U test is used for the test shown in Fig. 7b, with statistical significance of p<0.05.

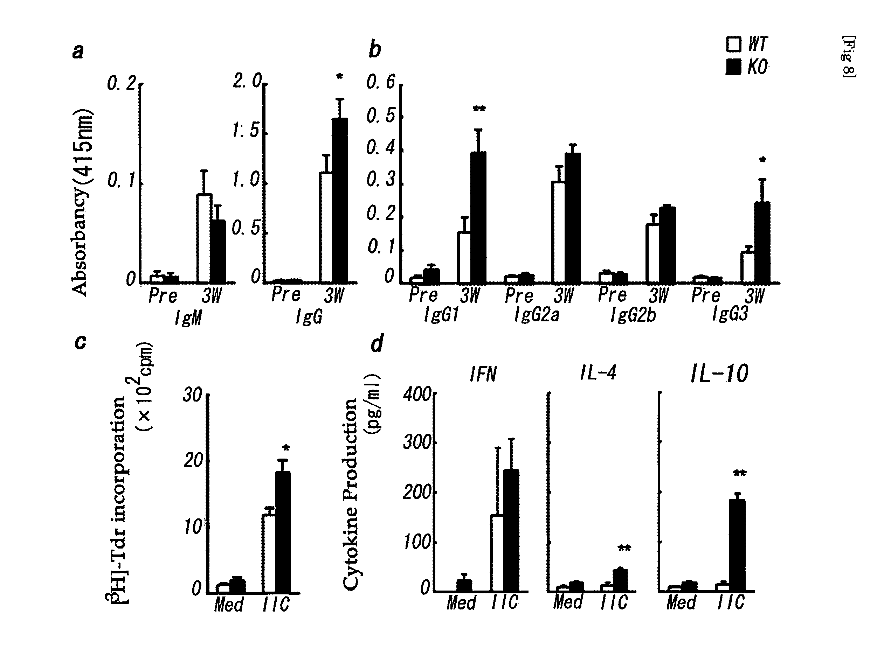

Figs. 8a to 8d are drawings that show elevated immune response in DCIR-/- mice when CIA was induced.
Fig. 8a is a graph that shows serum levels of IgM and IgG in a DCIR-/- mouse after IIC/CFA immunization.
Fig. 8b is a graph that shows levels of IIC-specific IgG subclasses. Serum of WT mice (WT, n=12) and DCIR-/- mice (KO, n=10) was collected before immunization (Pre) and 3 weeks after IIC/CFA immunization (3W), and level of IIC specific antibody was determined for each IgG subclass by ELISA.
Fig. 8c is a graph that shows proliferation response of IIC specific T cells. Lymph node cells were collected from WT mice (WT, n=9) and DCIR-/- mice (KO, n=9) 1 week after IIC/CFA immunization, cultured for 72h under absence (Med) or presence (CII) of 100mg/ml denatured chicken IIC to determine incorporation of tritium thymidine. The graph shows the pooled data of three independent experiments.
Fig. 8d shows a graph that shows cytokine production from lymph node cells. Levels of IFN-g, IL-4 and IL-10 were determined by ELISA in supernatant of lymph node cell culture of WT mice (WT, n=5) and DCIR-/- mice (KO, n=5) in the system shown in Fig. 8c. The graph shows mean and SEM. *P<0.05, ** p<0.005.

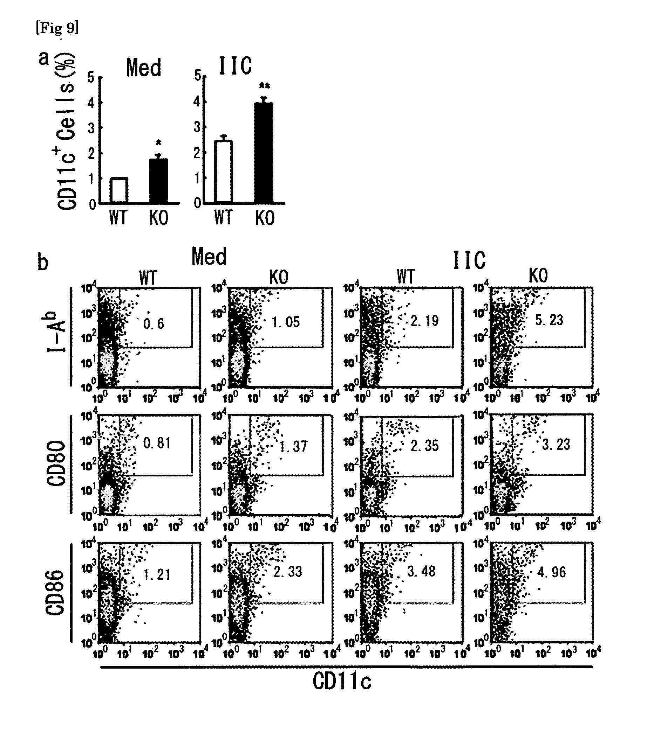

Figs. 9a and 9b are drawings that show elevated proliferation of dendritic cells in IIC/CFA immunized DCIR-/- mice. Lymph node cells were collected from WT mice (WT, n=3) and DCIR-/- mice (KO, n=3) 1 week after IIC/CFA immunization, and cultured for 72h under absence (Med) or presence (CII) of 100mg/ml denatured chicken IIC.
Fig. 9a is a graph that shows the result of FACS analysis of stained CD11c. The graph shows mean and SEM. * p<0.05, ** p<0.01
Fig. 9b is a drawing that shows the result of flow cytometry of lymph node cells which were cultured for 72h under absence (Med) or presence (CII) of denatured chicken IIC, then CD11c and I-A^{b}, CD80 or CD86 was double stained. The figures in the drawing show percentage of the cells in the gate.

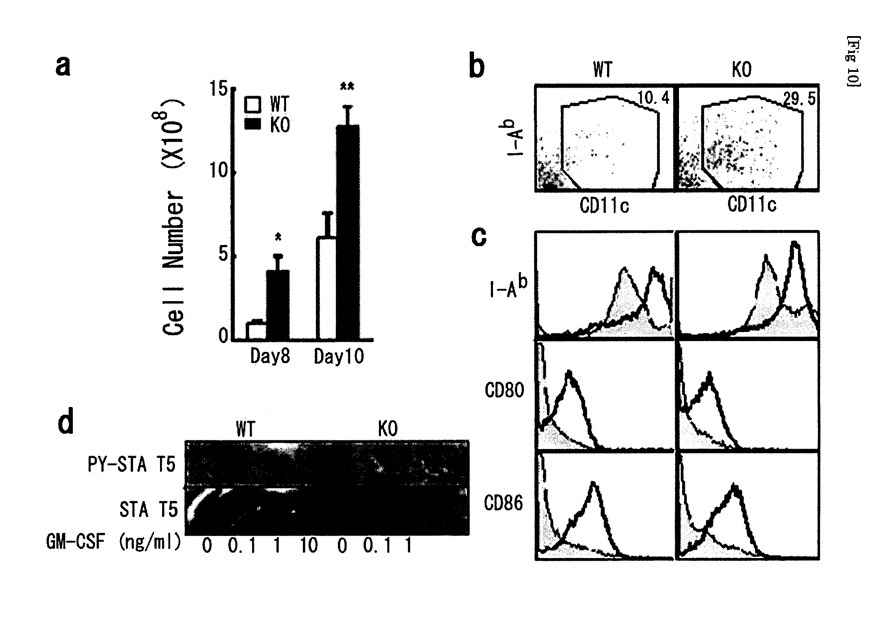

Figs. 10a to 10d are drawing that show elevated response of DCIR defective bone marrow cells (DCIR-/- BMC) to GM-CSF.
Fig. 10a is a graph that shows counts of cells derived from nonadhesive bone marrow determined at day 8 and day 10. The bone marrow cells derived from WT mice (WT) and DCIR-/- mice (KO) were cultured with GM-CSF added (n=4). * p<0.05, **p<0.01
Fig. 10b is a drawing that shows the result of flow cytometry of stained CD11c and I-A^{b} derived from nonadhesive bone marrow derived cells at day 8 of culture. The putative bone marrow derived dendritic cells are shown by the gate, and the figures show percentage of these cells to total cells.
Fig. 10c is a drawing that shows the result of flow cytometry of stained I-A^{b}, CD80 or CD86 of CD11c positive cells of bone marrow derived dendritic cells. The bone marrow derived dendritic cells were cultured for 48h under absence (shaded histogram) or presence (open histogram) of LPS, and flow cytometry was conducted at day 10 of culture.
Fig. 10d is a drawing that shows STAT5 and phosphorylated STAT5 in bone marrow cells detected by anti-STAT5 antibody and anti-phosphorylated STAT5 antibody. Bone marrow cells were treated with GM-CSF at concentration shown in the figure for 20 minutes to adjust total cell solution for electrophoresis.

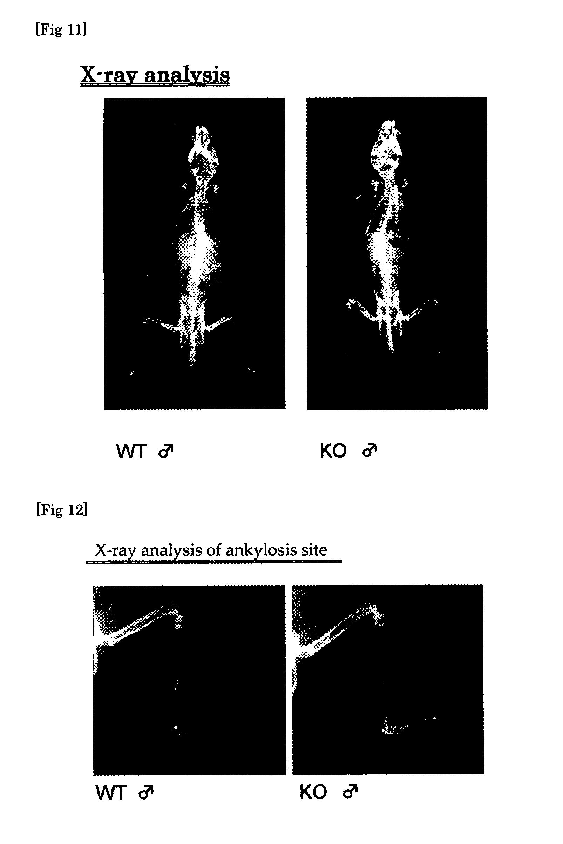

Fig. 11 is an X-ray image that shows skeleton of a DCIR-/- mouse and that of a WT mouse in Example 11.
Fig. 12 is an X-ray image that shows ankylosis site (heel) of a DCIR-/- mouse and a WT mouse in Example 11.

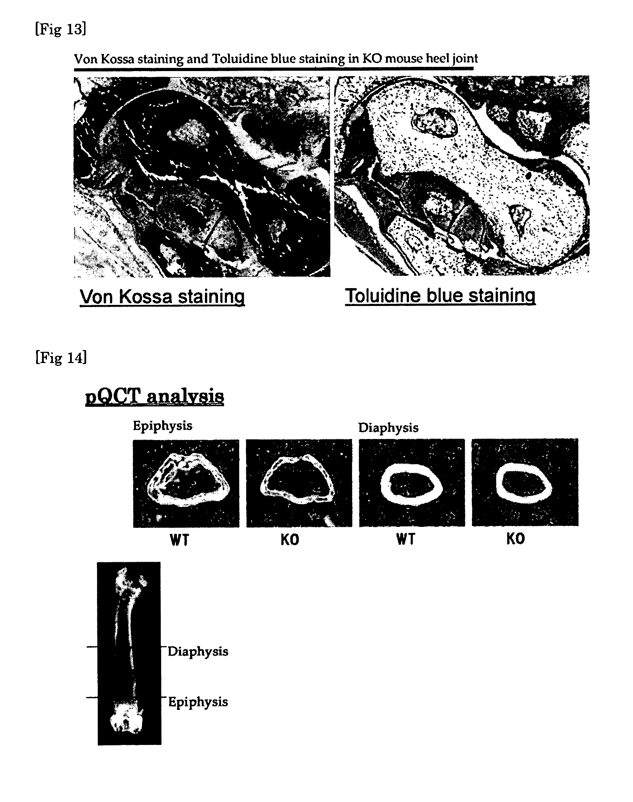

Fig. 13 is a cross-sectional view that shows the Von-Kossa strained (left) and Toluidine blue stained (right) heel joint of a DCIR-/- mouse in Example 11.
Fig. 14 is a photo that shows pQCT analysis of femur of a DCIR-/- mouse.

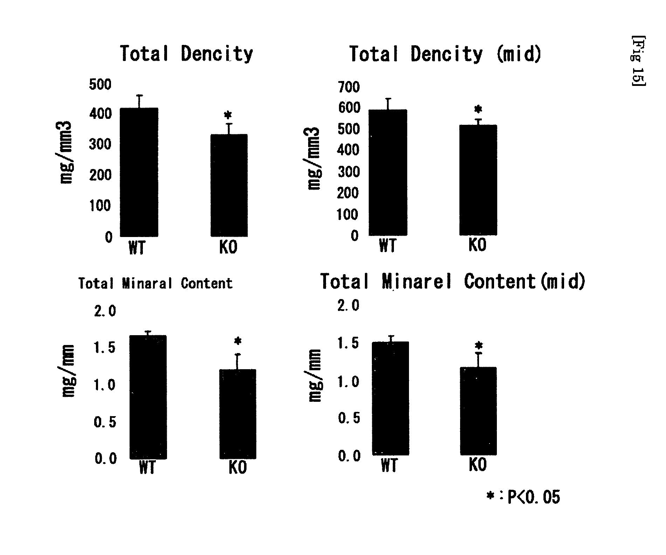

Fig. 15 is a graph that shows bone density and bone mineral of DCIR-/- mice and WT mice determined by pQCT analysis. The bone density is mean of selected regions, while bone mineral is mean of selected regions with thickness of 1mm.

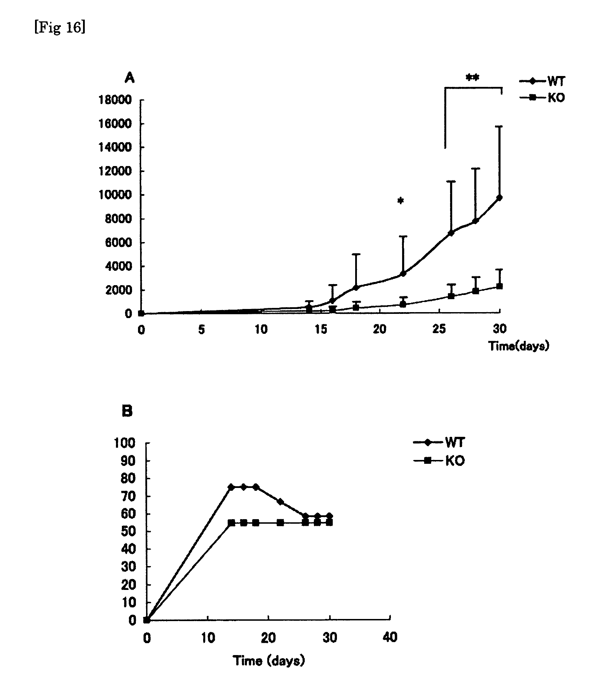

Fig. 16a is a graph that shows size of tumor and 16b is a graph that shows incidence of tumor, over time when DCIR-/- mice and WT mice were injected with MethA. WT: n=12, KO: n=11, ** <0.05, *<0.01

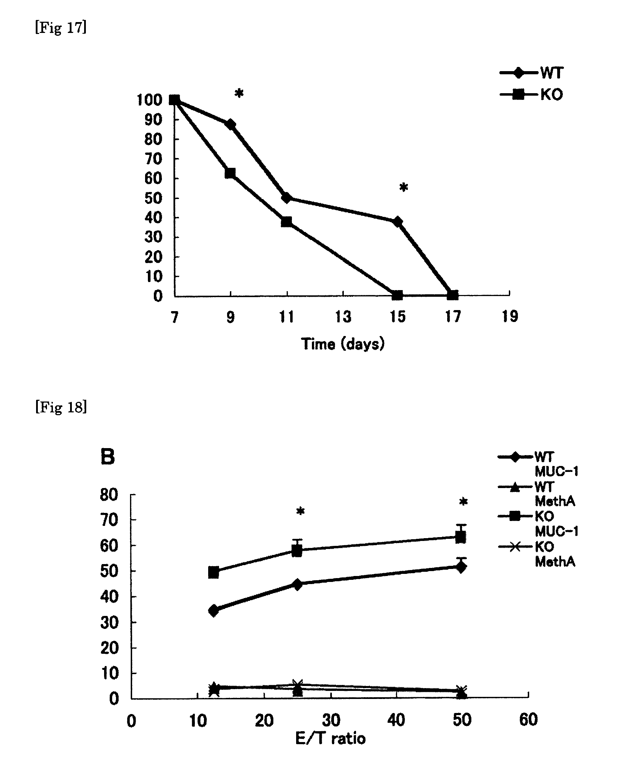

Fig. 17 is a graph that shows change in incidence in DCIR-/- mice and WT mice injected with BALB/3T3 APR-MUC1 clone16. n=8, *<0.05.
Fig. 18 is a graph that shows the cytotoxity determined by using BALB/3T3 APR-MUC1 clone 16. *<0.01

### Preferred embodiment of the invention

Preferably, the disease model animal according to the invention uses a rodent, more preferably, it uses a mouse. Since the model animal according to the invention spontaneously develops autoimmune diseases such as arthritis, it is useful as a model animal of autoimmune diseases. The autoimmune diseases that can be studied using the model animal according to the invention include rheumatoid arthritis (RA), ankylosing spondylitis (AS), Sjogren syndrome (SS) and diffused idiopathic skeletal hyperostosis (DISH), but not limited thereto. The mouse also can be used as a model animal of metabolic bone diseases such as osteoporosis, as well.

The screening method according to the invention comprises steps of administering a test substance to the disease model animal as above or exposing the tissue, organ or cell derived from the animal to the test substance, determining and evaluating the differentiation and proliferation of bone marrow stem cell derived cells in the animal or tissue, organ or cells thereof, and identifying a substance that facilitates or suppresses differentiation and proliferation of bone marrow stem cell derived cells. Alternatively, the method comprises steps of administering the test substance to the disease model animal, determining and evaluating incidence and score of collagen induced arthritis, and identifying a substance to suppress development of arthritis.

Preferably, the screening method according to the invention further comprises a step of comparing the result of determination and evaluation of the disease model animal with that of a wild type animal. The screening method as above can identify a substance effective for prevention and treatment of autoimmune diseases including RA, AS, psoriatic arthritis (PsA), SS and DISH, and osteoporosis.

As mentioned above, the disease model animal according to the invention (DCIR-/- mouse) spontaneously develops or aggravates autoimmune diseases. This finding indicates that DCIR protein is effective in prevention and treatment of the disease. Therefore, the invention provides a preventive/remedy for autoimmune diseases or osteoporosis, the preventive/remedy comprising a substance that facilitates expression of gene coding DCIR protein.
Furthermore, since the DCIR protein defective animal develops autoimmune diseases, a gene coding DCIR protein or a partial sequence thereof is effective as a diagnostic agent of autoimmune diseases or osteoporosis. The invention provides a diagnostic agent of autoimmune diseases or osteoporosis, comprising such DNA or partial sequence thereof.

A peptide having an amino acid sequence identical or substantially identical to DCIR protein, a partial peptide or a salt thereof can screen a substance that facilitates activity of DCIR protein, that is, a preventive/remedy for autoimmune diseases or osteoporosis, by co-culturing the peptide, the partial peptide or the salt thereof with a candidate substance, and comparing the activity of DCIR protein under presence or absence of the candidate substance. The invention provides a kit for screening such a preventive/remedy.

A base sequence identical or substantially identical to the gene coding DCIR protein, or partial sequence thereof, can be used as a probe to determine presence or absence and degree of expression of DNA coding DCIR and to determine susceptibility of a subject to autoimmune diseases or osteoporosis. The invention provides a kit for determining such susceptibility.

The inventors clarified for the first time that DCIR is involved in development or aggravation of autoimmune diseases. DCIR is one of inhibitory regulators in immune system, playing a role to suppress immune response under physiologic condition. Therefore, by administering a peptide comprising an amino acid sequence identical or substantially identical to DCIR protein (soluble extracellular protein moiety, in particular), or a partial peptide or salt thereof, inhibitory signals in immune system may be inhibited, activating immune system. Therefore, the peptide, the partial peptide or salt thereof can be used as a remedy for various infections caused by various viruses, bacteria, fungi or protozoa.
Recently, in cancer immunotherapy, a method comprising making dendritic cells intake tumor cells or antigen in vitro, activating and maturing the dendritic cells and administering the cells to a patient attracts attention as the method may activate immune response of the patient. This method has another advantage of lower risk of adverse effect because it uses dendritic cells derived from the patient himself. Therefore, the peptide of DCIA protein or equivalent thereof may be useful as an activating agent for cancer immunotherapy.

### Examples

Next, the invention will be described in detail with reference to specific embodiment thereof, but the invention is not limited to these embodiments.

### Experiment method

### Mouse:

Two mouse models, HTLV-I-Tg mice and IL-1 Ra-/- mice were used for gene expression profiling. HTLV-I-Tg mice were prepared by injecting LTR-env-pX-LTR region of HTLV-I genome to (C3H/Hen x C57BL/6J) F₁ embryos. The resulting mice were backcrossed for 20 generations to BALB/cA mice (CLEA Japan, Tokyo, Japan). The resulting mice spontaneously develop arthritis at 4 weeks of age, the incidence being 60% at 3 months of age, and 80% at 6 months of age. In the following examples, HTLV-I-Tg (TS) mice (female at 6 to 9 weeks of age) that developed severe arthritis (score 3) were used. IL-1Ra-/- mice prepared by homologous recombination were backcrossed for 8 generations to BALB/cA mice. These mice spontaneously develop arthritis at 5 weeks of age, the incidence being 80% at 8 weeks of age and 100% at 13 weeks of age. In the following examples, IL-1Ra-/- mice (KS) (male at 13 weeks of age) that developed severe arthritis (score 3) were used. Wild type litter mice (WT) were used as a control.

The severity of arthritis was scored from 0 to 3 based on the extent of redness and swelling of feet. Score 0 = normal, Score 1 = mild swelling in joint and/or redness in foot, Score 2 = marked swelling of joint, Score 3 = severe swelling and ankylosis of joint. All mice were bred under specific pathogen free (SPF) condition in a clean room of The Institute of Medical Science, The University of Tokyo. All experiments were conducted in conformity with ethical guidelines for animal experimentation and safety standard regarding to gene engineering.

Northern Blot Hybridization:
The joint tissue was immediately frozen in liquid nitrogen, and kept at -80°C. The frozen tissue was homogenized with physcotron (Microtech, Chiba, Japan). From the homogenate, total RNA was prepared using guanidine isothiocyanate phenol chloroform method, and poly (A)⁺ RNA was purified using oligo (dT) cellulose column. For each sample, RNA derived from 4 to 5 mice was pooled. Poly (A)⁺ RNA was electrophoresed in 1.3% denaturing agarose gel, and transferred to a nylon membrane (Gene Screen Plus, NEN Life Science, Boston, MA, USA). Hybridization was conducted using ³²P-labeled DNA probe labeled with Megaprime DNA labeling system (Amersham, Arlington Heights, IL, USA) and ³²P-dCTP (3,000 Ci/mmol: NEN Life Science, Boston, MA, USA) at 42°C over night. Radioactivity was determined by BAS-2000 system (Fuji Photo Film Co., Tokyo, Japan). Mouse DCIR probe was amplified from cDNA derived from joint of an HTLV-I-Tg mouse with arthritis, using PCR. The following PCR primers were used:
5'-CAT TTC CCT TAT CTC GCC CTG G-3' (SEQ NO: 4)
5'-GCA GCA TGA ATG TCC AAG ATC C-3' (SEQ NO: 5)

Preparation of DCIR-/- mouse:
The sequence of genome DNA containing DCIR was obtained from mouse genome database provided by Celera Genomics (Rockville, MD, USA), and a 5' homology region comprising a 5.5-kb fragment and a 3' homology region comprising a 2.5-kb fragment were amplified from genome DNA derived from ES cells (E14.1) by PCR. Following sets of PCR primers were used:
   5'-arm:
      5'-GAT TAAAAG CGG CCG CCA GAA TTC GTT TGA GAT CAG GC-3' (SEQ NO:6)
      5'-CTG GAT CCG TCA GAA GAG AGC CTT GTT CC-3'(SEQ NO: 7)
   3'-arm:
      5'-CCA TCG ATG AAG AGA GGT TCC ACT CTA GC-3' (SEQ NO: 8)
      5'-TTA TCG ATG TCAACT ACC TTT GCA TTG GG-3' (SEQ NO: 9)

Targeting vector was prepared by substituting a genome fragment coding the first and second exons containing the intracellular domain containing ITIM and the transmembrane domain, with a fragment expressing neomycin resistance gene (Neo) under control of PGK1 promoter. For negative selection, a fragment expressing diphtheria toxin (DT) under control of MC1 promoter was ligated to 3'-terminal. The targeting vector was introduced to ES cells by electoporation and the resultant cells were selected by G418. 672 neomycin resistant ES clones were picked up, from which 660 clones were screened by southern blot hybridization using 5' probe.
5' probe used for the screening was amplified using the following primers:
5'-TAA CAC TGA GGG AAG ATG CTA C-3' (SEQ NO: 10)
5'-TCT CAT TCT CAC TCT CAC TCT C-3' (SEQ NO: 11)

96.2% of the clones were identified by southern blot hybridization, and 2 targeting ES clones were identified (Targeting efficiency: 0.3%). These targeting clones were confirmed by 3'-probe and Neo probe.
3'-probe was amplified using the following primers:
5'-AGC CAT GAT AAC AGA CCC-3' (SEQ NO: 12)
5'-TGA TAT GGG GTC TGG TAC G-3' (SEQ NO: 13)
Narl-Xbal fragment of neomycin resistant gene was used as Neo probe. Karyotyping showed that about 80% of the cells of both clones has normal 40 chromosomes. Chimera mice were prepared by aggregation method. A male chimera mouse was mated with a wild type C57BL/6J female mouse, and transmission to the germ cells was determined by fur color. Heterozygosis mice in terms of DCIR mutation were mated to prepare homozygosis mice. In the following examples, DCIR-/- mice and its litter mice with (129/Sv x C57BU6) F₁ genetic background were used.
Genotyping of DCIR-/- mouse used the following PCR primers:
Common primer: 5'-AAG TGT CCC CTC TTG TAC TCT GTG-3 (SEQ NO: 14)
Wild type primer: 5'- CAAAAT TCT GTC AAG CGT AGA GGG G-3' (SEQ NO: 15)
Mutant primer: 5'-CAT TAT ACG AAG TTA TCT CGA GTC GC-3' (SEQ NO: 16)
   The common primer and the wild type primer were used to detect wild type allele (1.3 kb) and the common primer and the mutant primer were used to detect mutant allele (0.9 kb).

Histopathology:
A WT mouse and a DCIR-/- mouse at 12 months of age were anesthetized with ether, and perfusion fixed with neutral buffer 10% formalin solution. Major organs and tissues including brain, heart, aorta, lung, bronchium, pancreas, liver, spleen, axillary lymph node, submaxillary gland and parotid gland, intestine, stomach, kidney and reproductive tract were excised for histopathologic observation. Joint tissues including foot, knee, wrist and thoracic vertebra were decalcified using 10% formic acid and paraffin embedded. For each tissue, 2 to 3 mm sections were prepared and stained with hematoxylin and eosin.

Flow cytometry:
Cells were stained with following monoclonal antibodies (mAb) conjugated with FITC, PE or biotin, and used for flow cytometry. mAb of a hamster, a mouse or a rat specific to CD11c (HL3), I-A^{b} (AF6-120.1), CD3 (145-2C11), CD45R/B220 (RA3-6B2), CD4 (RM4-5), CD8 (53-6.7), CD62L (MEL-14) or CD44 (IM-7) were purchased from BD PharMingen (San Diego, CA, USA). Rat mAb specific to CD80 (RMMP2), CD86 (RMMP1) of a mouse was purchased from Immunotech (Marseille, France). PE conjugated StreptAvidin (PharMingen) was used for secondary staining of biotin conjugated antibody. The surface of cells were stained using standard protocol, and analysed using FACSCalibur and CellQuest (Becton Dickenson, Franklin Lakes, NJ, USA) or FlowJo (Tree Star, Inc. Ashland, OR) software.

Proliferation:
In order to investigate antigen specific response, Thy 1.2 positive T cells were prepared from a WT mouse, CD11c positive dendritic cells were purified from spleens of a WT mouse and a DCIR-/- mouse by magnetic cell sorting using CD90 (Thy1.2) microbeads or CD11c microbeads (MiltenyiBiotech, Bergisch Gladbach, Germany). Purified Thy1.2 positive T cells (2 x 10⁵ cells) and
CD11c positive dendritic cells (2x10⁴ cells) were co-cultured for 3 days with anti-CD3 antibody (145-2C11: BD PharMingen), and left to incorporate tritium thymidine (0.25 µ Ci/ml: Amersham, Buckinghamshire England) for 6 hours. Next, the cells were collected on a Micro 96 cell harvester (Skatron, Lier, Norway) glass fiber filter, and radioactivity of tritium thymidine was determined using Micro Beta (Pharmacia Biotech, Piscataway, NJ).
In order to investigate allogeneic mixed leucocyte reaction, Thy1.2 positive T cells were purified from a BALB/c mouse by magnetic cell sorting using CD90 (Thy1.2) microbeads. Dendritic cells were induced from bone marrow cells of a WT mouse and a DCIR-/- mouse. The dendritic cells were treated with mitomycin C, co-cultured with Thy1.2 positive T cells for 3 days, and left to incorporate tritium thymidine for 6 hours to determine radioactivity thereof.

Collagen induced arthritis:
To complete Freund's adjuvant (Difco Laboratories Detroit, MI) added with 5mg/ml heat killed M tuberculosis (H37RA: Difco), 1mg/ml chicken II type collagen (IIC: Sigma-Aldrich, St. Louse, MO) was suspended, and the suspension was immunized to several subcutaneous sites of the tails of the mice. Arthritis was induced without additional immunization. Their joints were observed as to presence of swelling and redness for several days, and if any swelling or redness were observed, its severity was scored to 0 to 3. (Score 0= normal: Score 1= mild swelling of joint and/or redness on foot: score 2 = marked swelling of joint: Score 3 = severe swelling and ankylosis of joint). For histological evaluation of arthritis, the joint tissue was fixed in 10% neutral buffer formalin solution, decalcified with 5% formic acid, and paraffin-embedded. From tissues, 4mm sections were prepared and stained with hematoxylin and eosin.

Determination of antibody:
10mg/ml chicken IIC for determining collagen specific antibody; rabbit anti mouse IgM (2mg/ml: Zymed), IgG (8.7mg/ml: Zymed) or IgE (2mg/ml: PharmMingen) for determining serum antibody level; heat-denatured rabbit IgM, IgG (50mg/ml: Zymed) or chicken IIC (20mg/ml: Sigma) for determining autoantibody, were coated to Falcon 3912 Micro Test III flexible Assay plate (BD Bioscience. Oxnard, CA) overnight at 4°C. After washed with PBS, diluted serum samples were incubated for 1 hour at room temperature. After washed with PBS-Tween, alkaline phosphatase (AP) conjugated goat anti-mouse IgM, IgG, IgE, IgG1, IgG2a, IgG2b and IgG4 were incubated for 1 hour at room temperature, and AP activity was determined by ELISA microreader (MTP-120: Colona, Tokyo, Japan) using Substrate Phosphatase SIGMA104 (Sigma-Aldrich). For determining antinuclear antibody, diluted mouse serum was incubated on mouse nucleus antigen coating plate (Alpha Diagnostic, Inc., San Antonio, TX), reacted with horseradish peroxidase (HRP) conjugated goat anti mouse IgG, and HRP activity was determined using TMB substrate.

Collagen specific proliferation reaction and cytokine production:
Lymph nodes were collected 7 days after IIC/CFA immunization to prepare single cell suspension. Lymph node cells were incubated under presence or absence of heat-denatured IIC for 3 days, left to incorporate tritium thymidine (0.25mCi/ml) for 6 hours to determine tritium thymidine radioactivity. For determining cytokine production, culture supernatant was collected 3 days after collagen specific proliferation reaction, and IFN-γ, IL-4 and IL-10 levels were determined using BD OptiEIA ELISA Set (BD PharMingen).

Preparation of bone marrow cell derived dendritic cells
Bone marrow cell derived dendritic cells were prepared from bone marrow of femur and tibia. Briefly, 2x10⁵ cells/ml of bone marrow cells were cultured in 10% FCS-RPMI-1640 added with 20ng/ml recombinant mouse GM-CSF (PeproTech, London, UK). At day 3, double dose of fresh medium containing 20ng/ml GM-CSF was added, and at day 6 and 8, half of medium supernatant was collected for centrifugation. The cells were re-suspended in a fresh medium containing 10ng/ml GM-CSF and returned to the original plate. Non-adhesive cells at day 10 were used as bone marrow cell derived dendritic cells. Furthermore, for complete maturation of bone marrow cell derived dendritic cells, non-adhesive cells at day 10 were collected, and re-suspended in a fresh medium containing 10ng/ml GM-CSF and 1mg/ml LPS (Sigma). These cells were incubated for additional 2 days, and used as matured dendritic cells.

Western blotting:
Bone marrow cells were prepared from femur and tibia, and cultured overnight on nonserum RPMI-1640 medium added with 20ng/ml recombinant mouse GM-CSF. The cells were washed and resuspended in nonserum medium without GM-CSF. Cells were left starved for 6 hours, treated with GM-CSF and phosphatase inhibitor cocktail II (Sigma) at various concentration. Total cell solution was analyzed by immuno blotting using anti-phosphorylated STAT 5 antibody (Cell Signaling, Denvers, MA) and anti STAT5 antibody (Santa Cruz Biotechnology, Inc., Santa Cruz, CA). Horseradish peroxidase (HRP) conjugated anti rabbit IgG (Cell Signaling) was used as detection antibody. Luminescent signal was detected using ECL plus detection solution and Typhoon 9000 (Amersham Biosciences, Buckinghamshire, UK).

Statistics:
For statistical analysis, Student t-test was used except followings: _{X}2 test was used for incidence of CIA experiment; Mann-Whitney U-test was used for severity, and incidence of hisopathological abnormality.

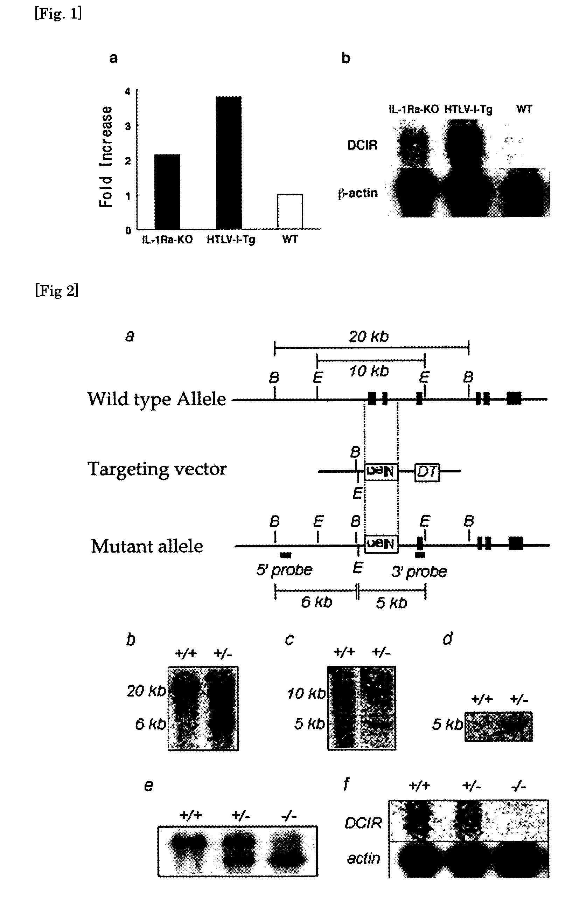

(Example 1)
Identification of DCIR as RA related gene:
Gene expression profile was compared between joints of two types of model mouse (HTLV-I Tg and IL-1Ra-/- mouse) and that of a normal mouse to identity gene showing elevated expression in joints with arthritis. Microarray analysis revealed mRNA expression of DCIR gene was 2.2 times in an IL-1 Ra-/- mouse, and 3.8 times in an HTLV-I Tg mouse, compared to that in a WT mouse (Fig. 1a). This elevated expression was also confirmed by northern blot hybridization (Fig. 1b).

(Example 2)
Preparation of DCIR-/- mouse:
A DCIR-/- mouse was prepared by conventional gene targeting method. Exons 1 and 2 of DCIR gene were substituted with neomycin resistant gene to delete genome sequence that codes major part of cytoplasmic domain including ITIM and transmembrane domain (Fig. 2a). Targeting ES clone was screened by southern blot hybridization using 5' probe (Fig. 2b), and targeting allele was identified by 3' probe (Fig. 2c) and Neo probe (Fig 2d). Targeting of DCIR domain in a mouse was confirmed by genome southern blotting (Fig. 2e). DCIR-/- mice was prepared by mating DCR+/-mice, and it was confirmed that the resultant DCIR-/- mice lacked expression of DCIR mRNA in the spleen by northern blot hybridization (Fig. 2f).

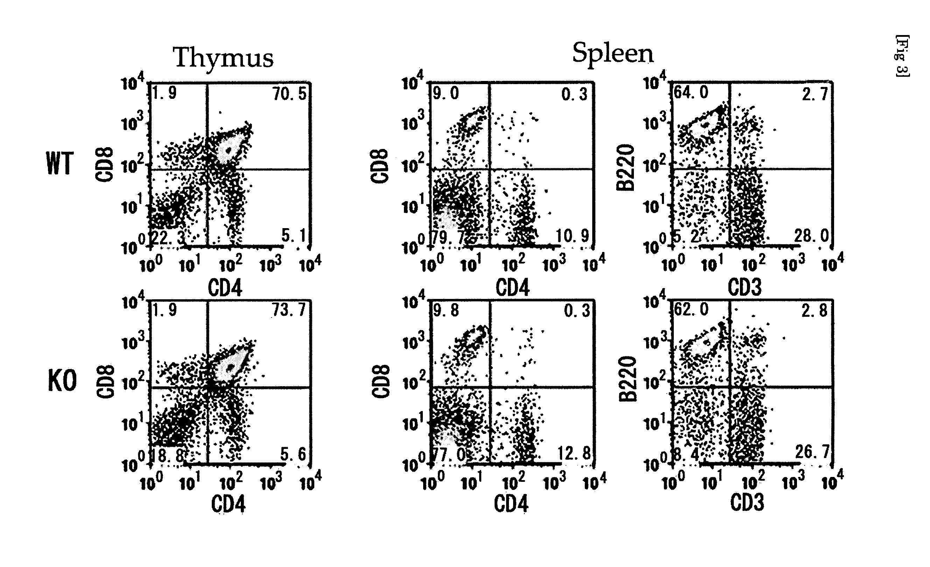

DCIR-/- mice were fertile, and their offspring showed Mendelian inheritance. Young DCIR-/- mice showed no aberrant phenotype under specific pathogen free condition. No abnormality was observed in spleen and thymus cells of DCIR-/- mice (Fig. 3). In order to determine effect of DCIR deficiency on T cell response, proliferation and allogeneic mixed lymphocyte reaction (MLR) of T cells induced by stimulation by anti CD3 antibody was determined under presence of dendritic cells. The result showed no significant difference between DCIR defective dendritic cells and wild type dendritic cells (Fig. 4).

(Example 3)
Spontaneous development of autoimmune diseases in aged DCIR-/- mice:
Histology of DCIR-/- mice revealed accumulation of lymphocytes in salivary gland interstitium, infiltration of monocytes into epithelium of minor conduit and associated sialadenitis characterized by damage to minor conduit, in submaxillary glands of 7 in 10 DCIR-/- mice at 6 to 12 months of age (Table 1).

At 4 months of age, 11% of DCIR-/- mice showed abnormal joints in their hind legs. Initially, the mice showed arthritis with redness and swelling in a plurality of joints, and by 6 months of age, 28% of mice developed arthritis. Incidence and severity of arthritis were worsened over time, bringing about deformation and ankylosis of joints. The symptoms were marked in joints in hind legs. Incidence showed sex difference: the percentage of mice that developed symptom was 44% in the male mice while 6% in the female mice at 12 months of age. Histological observation of joints of DCIR-/- mice revealed infiltration of inflammatory cells to insertion of tendon and ligament to bone, and destruction of bone by invading granulated tissue. Enthesitis was present in 9 in 23 DCIR-/- mice observed (Table 1). These findings indicate that deficiency of DCIR gene induces autoimmune, and spontaneous development of delayed arthritis and sialadenitis.

**Table 1**

| Incidence of histopathological abnormality in aged DCIR-/- mouse | | | | |
|---|---|---|---|---|
| Symptom | Genotype | Number of mouse with symptom/total mouse | | |
| | | | (%) | |
| | | Male | Female | Total |
| Sialadenitis | Wild type | 0/3 (0) | 0/2 (0) | 0/5 (0) |
| | DCIR-/- | 4/6 (67)* | 3/4 (75)* | 7/10 (70)** |
| | | | | |
| Enthesitis | Wild type | 0/5 (0) | - | 0/5 (0) |
| | DCIR-/- | 7/17 (41)* | 2/6 (33)* | 9/23 (39)* |

| | | | | |
|---|---|---|---|---|
| The table shows number of mice and incidence (figures in parenthesis) of sialadenitis and enthesitis in mice at 6 to 12 months of age * P<0.05, **p<0.01 (Mann-Whitney U test) | | | | |

(Example 4)
Spontaneous development of autoimmune in DCIR-/- mice:
Since it was indicated that DCIR deficiency influenced development of autoimmune, production of autoantibody in DCIR-/- mice was determined by ELISA. Serum levels of IgM, IgG, IgE and production of autoantibodies such as antinuclear antibody (ANA), rheumatoid factor (RF: IgM and IgG types), and anti-IIC IgG were determined (Fig. 5). There was an increasing tendency in serum immune globulin level of DCIR-/- mice at 12 months of age, without significant difference except IgE level. Aged DCIR-/- mice showed increased production of autoantibodies such as antinuclear antibody (ANA), rheumatoid factor (RF) and anti-IIC antibody, compared to WT mice. The findings indicate that DCIR deficiency relates to production of autoantibodies.

(Example 5)
Proliferation of dendritic cells and activation of CD4 positive T cells in a DCIR-/- mouse:
The ratio of CD11c positive cells that expressed DCIR was determined in a DCIR-/- mouse. A young mouse showed no abnormality in the ratio under physiological condition, but a mouse at 12 or more months of age showed marked increase in the ratio. Most of CD11c positive cells expressed I-A^{b}. CD4 positive T cells of a DCIR-/- mouse had lower expression of CD62L that is a characteristic of activated T cells, but higher expression of CD44. CD8 positive T cells showed no marked change (Fig. 6).

(Example 6)
Aggravation of collagen induced arthritis in a DCIR-/- mouse
Since expression of DCIR was increased in the RA model mouse according to the invention, the effect of DCIR deficiency on development of collagen induced arthritis (CIA) was tested. CIA was induced by immunizing 250mg of complete Freund's adjuvant added with heat-killed Mycobacterium tuberculosis, and 100mg of chicken type II collagen (IIC) to young DCIR-/- mice with (129.Sv x C57BU6) F₁ hybrid background. In a conventional protocol, additional immunization with IIC/CFA is to be conducted at day 21 after the first immunization. However, in this experiment, 20 days after first immunization, 0% of litter and 39% of DCIR-/- mice developed symptom (Fig. 7). When additional immunization is used, it might be difficult to observe increase in incidence in DCIR-/- mice, so that the observation was continued without additional immunization. As shown in Fig. 7, the incidence of arthritis in DCIR-/- mice was markedly higher than that in the litter as control. Also, the DCIR-/- mice developed significantly severer symptom compared to WT mice. The incidence and severity in the groups are accumulated data of two independent experiments.

Histology of CIA in the joints of control mice revealed minimum cell infiltration with no proliferated synovial membrane or destroyed bone. On the other hand, DCIR-/- mice developed typical arthritis with infiltration of inflammatory cells, proliferation of synovial membrane and destruction of bone even under this mild immunization. The findings indicate that DCIR suppresses development of CIA.

(Example 7)
Increased production of antigen specific IgG1 and IgG3 antibodies in DCIR-/- mice
Next, production of IIC specific antibody in DCIR-/- mice immunized with IIC was determined. It is known that level of antibody against IIC is well-correlated to development of arthritis. As shown in Figs. 8a and 8b, levels of IgG, IgG1 and IgG3 specific to IIC were significantly higher in DCIR-/- mice than in the wild type litters. On the other hand, levels of IgM, IgG2a and IgG2b in DCIR-/- mice showed no abnormality. These findings indicate involvement of DCIR in production of IgG1 and IgG3 subclass antibodies specific to antigen in CIA.

(Example 8)
Elevated antigen specific proliferation of T cells in DCIR-/- mice
Antigen specific proliferation of T cells derived from DCIR-/- mice and control litters was tested. Reaction of T cells to IIC was determined 7 days after initial immunization with chicken IIC/CFA. Antigen specific proliferation of T cells was markedly higher in DCIR-/- mice than in WT mice (Fig. 8c), indicating that priming of T cells was facilitated in DCIR-/- mice. Production of IFN-γ from lymph node cells after stimulation with IIC was observed similarly in DCIR-/- mice and control mice. On the other hand, levels of IL-4 and IL-10 were significantly higher in DCIR defective lymph node cells (Fig. 8d). These findings indicate that production of cytokine from Th2 was selectively facilitated in a DCIR-/- mouse, consistent with observation of facilitated production of IIC specific IgG1 and IgG3 subclass antibodies.

(Example 9)
Determination of content and activity of DC after IIC/CFA immunization revealed marked increase in the ratio of CD11c positive cells in lymph node of DCIR-/- mice. Furthermore, the ratio of activated dendritic cells that expresses I-A^{b}, CD80 and CD86 was markedly higher in DCIR-/- mice than in WT mice. These findings demonstrate that differentiation and activation of dendritic cells in a DCIR-/- mouse is facilitated by IIC/CFA immunization.

(Example 10)
Elevated reaction of DCIR defective bone marrow cells (BMC) to GM-CSF
Since DCIR expresses mainly on dendritic cells, differentiation of DCIR defective bone marrow cells to dendritic cells was determined. When DCIR defective bone marrow cells were cultured with GM-CSF, differentiation of non-adhesive bone marrow derived cells was significantly higher in DCIR defective bone marrow cells (Fig. 10a). Flow cytometry of non-adhesive cells at day 8 of incubation showed marked increase of CD11c positive bone marrow cells derived dendritic cells (BMDC) in the culture of DCIR defective bone marrow cells (Fig. 10b). Most of these CD11 c positive cells expressed I-A^{b}, without expression of activated markers CD80 and CD86. When BMDC at day 10 of culture was subject to LPS pulse for 48 hours, the dendritic cells derived from a DCIR-/- mouse and a WT mouse showed marked expression of I-A^{b}, CD80 and CD86, indicating similar level of maturation of both types of cells (Fig. 10c).

Next, the phosphorylation of STAT5 induced by GM-CSF stimulus in bone marrow cells derived from a DCIR-/- mouse was analyzed. As shown in Fig 10d, activation of STAT 5 induced by GM-CSF was markedly increased in DCI R defective bone marrow cells, consistent with elevated reactivity of DCIR defective bone marrow cells to GM-CSF. Therefore, elevated differentiation of DCIR defective bone marrow cells to dendritic cells in vitro may be caused by increased sensitivity of bone marrow cells to GM-CSF signal. Consistent with this observation, the ratio of dendritic cells in vivo was normal in a young mouse under physiological condition, but increased over time or by immunization.

As described above, the invention provides a DCIR-/- mouse and indicates involvement of DCIR in autoimmune sialadenitis and arthritis.

The invention demonstrates that aged DCIR-/- mouse spontaneously develops characteristic autoimmune disease. DCIR-/- mice at 12 months of age showed increased serum immune globulin level, as well as elevated production of autoantibodies such as antinuclear antibody, rheumatoid factor and anti IIC antibody, compared to WT mice. Histologically, pathological change was observed in salivary gland and insertion of a DCIR-/- mouse. Infiltration of lymphocyte into salivary gland is a typical sign of Sjogren syndrome (SS), a representative chronic autoimmune disease affecting salivary gland. Several mouse strains such as NOD, MRU/Ipr and NZB/W F1 are widely accepted as SS model animal. Human patients with other autoimmune diseases including RA, mixed connective tissue disease (MCTD), ankylosing spondylitis (AS) and spondylarthritis (SpA) develop localized sialadenitis at high incidence, the development of which strongly correlates to production of rheumatoid factor. These findings indicate that DCIR plays an important role in stable function of immune system and that deficiency thereof induces autoimmune diseases.

Aged DCIR-/- mice also spontaneously develop delayed ankylosing arthropathy. Histopathology of these mice revealed erosive destruction of bone and insertion associated with infiltration of inflammatory cells. However, histopathology of DCIR-/- mouse tissue was evidently different from that of other types of RA model mice such as a HTLV-I-Tg mouse, a IL-1 Ra-KO mouse and a CIA mouse. Unlike other model mice that develop characteristic synovitis similar to human RA, enthesitis was the primary abnormality in the DCIR-/- mouse and synovitis was rarely observed. Synovitis is a characteristic sign of RA, while enthesitis is characteristic in inflammatory rheumatoid diseases such as AS, SpA and psoriatic arthritis (PsA). Therefore, DCIR deficiency may induce characteristic inflammatory arthritis similar to AS, SpA and PsA.

The invention indicates that CIA is aggravated in a DCIR-/- mouse. After immunization with II type collagen, serum level of antigen specific IgG1 and IgG3 was markedly increased in a DCIR-/- mouse. Also, elevated antigen specific T cells response and immune response to IIC were observed in a DCIR-/- mouse. The ratio of dendritic cells in these mice was markedly higher and they were activated. Increased ratio of dendritic cells was observed in an aged DCIR-/- mouse, but not in a young DCIR-/- mouse under physiological condition. Dendritic cells present antigen on the cell surface and produce a variety of immune regulating cytokines, playing an important role in activation of T cells. The findings of the invention indicate that elevated function of dendritic cells in a DCIR-/- mouse triggers autoimmune.

Since dendritic cells differentiate from bone marrow stem cells by GM-CSF, GM-CSF produced by stimulation of IIC/CFA may be involved in proliferation of DC in CIA. The invention demonstrates that the elevated sensitivity of GM-CSF signal transduction induced by DCIR deficiency facilitates proliferation and differentiation of bone marrow derived dendritic cells in vitro (Fig. 10). Elevated phosphorylation of STAT5 induced by GM-CSF stimulation in DCIR defective bone marrow cells indicates inhibitory regulation of DCIR over GM-CSF signal transduction. It is reported that human DCIR recruits SHP-1 and SHP-2. SHP-1 is tyrosine phosphatase containing SH2 domains and negatively controls cytokine signal transduction including GM-CSF. Furthermore, a motheaten mouse defective in SHP-1 develops systemic autoimmune and severe inflammation. Since ITIM of mouse DCIR is functional and its amino acid sequence is identical to that of ITIM of human DCIR, it can be inferred that mouse DCIR recruits SHP-1 and SHP-2 to regulate GM-CSF signal transduction.

It was demonstrated that the production of IIC specific antibodies such as IgG1 and IgG3 subclasses was elevated in a DCIR-/- mouse with CIA. It is reported that in a DBA/1 strain mouse, collagen specific IgG2a class antibody is involved in development of CIA, but the level of antigen specific IgG2a antibody (129xB6) did not significantly increase in a DCIR-/- mouse with (129xB6) F1 hybrid background. A DCIR-/- mouse showed significantly increased production of IL-4 and IL-10 which are known to be involved in class switch of IgG1 and IgG3. Based on these findings, it is indicated that DCIR is involved in regulation of Th2 response.

Dendritic cells are involved in differentiation of Th1 and Th2 effector T cells. Dendritic cells exposed to intracellular parasitic pathogen facilitate Th1 response, and some kinds of parasites facilitate differentiation of Th2 cells by dendritic cells. It is known that differentiation of regulating T cells (Treg) also is regulated by dendritic cells, and that specific pathogen induces production of Treg. Therefore, interaction between dendritic cells and pathogen may play an important role to induce specific subset of effector T cells, and DCIR may be one of receptors on dendritic cells involving in differentiation of specific T cells.

Recent exhaustive genome-wide linkage analyses have revealed many regions sensitive to autoimmune diseases. Particularly, mouse 6F2 site band which has DCIR gene, and rat 4q42 and human 12p13 region on same chromosome relate to several inflammatory diseases such as arthritis, systemic lupus erythematosus (SLE), spontaneous diabetes mellitus, atherosclerosis, encephalomyelitis, asthma, respiratory tract hypersensitivity and allergy. DCIR deficiency triggers autoimmune-like disease. Thus, it is indicated that DCIR may be one of sensitivity genes of inflammatory diseases.

(Example 11)
Change in bone metabolism in DCIR-/- mouse:
In order to investigate role of DCIR in bone metabolism, X-ray, observation of tissue section and pQCT analysis were conducted using a DCIR-/- mouse at 12 months of age.
In a DCIR -/- mouse, only male mouse develops ankylosis at heel joint.
X-ray of a DCIR-/- mouse and a WT mouse showed no difference in their skeleton, as shown in Fig. 11, demonstrating no problem in formation of skeleton.
On the other hand, X-ray revealed abnormality in the site of ankylosis (heel) developed in a DCIR-/- mouse. As shown in Fig. 12, the mouse had destroyed joint and increased calcification region at heel joint.

In order to further characterize the calcification region of the heel joint of a DCIR-/- mouse (KO), Von Kossa staining and Toluidine blue staining was used to investigate its cartilage tissue. As a result, proliferation of joint cartilage was observed in heel joint, with the cartilage replaced with bone (Fig. 13).
Next, in order to detect any abnormality in bone mass of femur, pQCT was conducted on an animal at 12 months of age (Fig. 14). The results were decreased bone density and bone mineral, that is, decreased bone mass (Fig. 15).
In summary, effects of DCIR gene include increased cartilage cells and ossified and calcified region in heel joint, and decreased bone mass in femur. It was indicated that DCIR may play a role in bone metabolism, regulation of differentiation and proliferation of cartilage cells, osteoblast and osteoclast cells. Based on these findings, a DCIR-/- mouse can serve as a good model mouse of osteoporosis, as well as AS and DISH.

Determination of antitumor immune effect in DCIR-/- mice:
(1) Evaluation of antitumor immune effect in transplanted cancer experiment Possible improvement in antitumor immune effect by DCIR deficiency as an inhibitory receptor was investigated using transplantable tumor cells.
   First, MethA was used as transplantable tumor cells. MethA was transplanted at dose of 1 x 10⁶ cell/head in DCIR-/- mice and WT mice, and difference in tumor formation was observed. There was no significant difference in incidence, but tumor mass was significantly lower in DCIR-/- mice than in WT mice (Fig. 16).
   Next, BALB/3T3 APR-MUC1 (BALB/c background) which human MUC1 known as cancer antigen was expressed on BALB/3T3 as fibrosarcoma was used as transplantable tumor cells. BALB/3T3 APR-MUC1 clone16 supplied from Institute of Development, Aging and Cancer, Tohoku University was cultured in RPM1-1640(SIGMA) containing 10% FBS (SIGMA), 50U penicillin (Meiji Seika Kaisha Ltd.), 50mg/ml streptomycin (Meiji Seika), and 500mg/ml G418 (Nacalai Tesque), in subculture method at frequency of 2 to 3 a week.
   BALB-3T3 APR-MUC1 clone 16 was injected at dose of 1 x 10⁶ cell/head, and a palpable tumor (about 4mm) was formed. Anti tumor immune effect was evaluated in terms of this incidence of tumor, and DCIR -/- mice showed significant reduction of tumor (Fig. 17).

(2) Evaluation of cytotoxic activity of dendritic cells in DCIR-/- mice
Induction of cytotoxic T cells (CTL) using tumor cell strain BALB/3T3 APR-MUC1 clone 16 was conducted as follows. Tumor cells were inactivated by 8000 rad γ-ray irradiation, and dosed intraperitoneally in mice at dose of 1 x 10⁷ cells/head at day 1 and 10. The spleen of the mice was collected 5 days after immunization to prepare spleen cells. The spleen cells were co-cultured with inactivated tumor cells on a 10-well plate in a ratio of 1x10⁷:1x10⁶, and stimulation was repeated for 3 days.
Target cells were labeled with 100µ Ci sodium chromate, ⁵¹Cr (GE Healthcare bioscience) at 37°C for 1 hour, and suspended in RPMI-1640 (10% FBS, 10mM 2-Mercaptoethanol). Lymphocytes were isolated from re-stimulated spleen cells by Lymphocyte-M (CEDARLANE), and co-cultured with the labeled target cells on a 96-well round-bottom plate (IWAKI). After 4 hours, emitted ⁵¹Cr was detected as radioactivity by Micro Bete (Pharmacia) to determine cytotoxic activity.

Cytotoxic activity was determined using BALB/3T3 APR-MUC1 clone 16 to find out elevated activity in DCIR-/- mice. Damage to MethA as a negative control was not observed (Fig. 18). The finding demonstrates significant elevation in specific cytotoxic activity in DCIR-/- mice.

## Claims

1. A nonhuman disease model animal **characterized by** having a partial or entire deficiency of gene coding dendritic cell immune receptor (DCIR) protein on chromosome.

2. The nonhuman disease model animal of Claim 1 wherein the animal is a rodent.

3. The nonhuman disease model animal of Claim 2 wherein the rodent is a mouse.

4. The nonhuman disease model animal of any of Claims 1 to 3 wherein the disease is an autoimmune disease.

5. The nonhuman disease model animal of Claim 4 wherein the disease is rheumatoid arthritis (RA), ankylosing spondylitis (AS), psoriatic arthritis (PsA), Sjogren syndrome (SS), or diffused idiopathic skeletal hyperostosis (DISH).

6. The nonhuman disease model animal of any of Claims 1 to 3 wherein the disease is osteoporosis.

7. A method to screen a substance that facilitates or suppresses differentiation and proliferation of bone marrow stem cell derived cells, comprising steps of administering a test substance to the nonhuman disease model animal of any of Claim 1 to 6, or exposing cells, tissues or organ derived from the animal to the test substance, and determining and evaluating differentiation and proliferation of bone marrow stem cell derived cells in the animal, or cells, tissues or organs thereof.

8. A method to screen a substance that suppresses development of arthritis, arthropathy or osteoporosis comprising steps of administering a test substance to the nonhuman disease model animal of any of Claims 1 to 4, and determining and evaluating incidence and severity of arthritis, arthropathy or osteoporosis in the animal.

9. The method to screen of any of Claims 7 and 8 comprising further step of, comparing the result of the determination and evaluation of the disease model animal of any of Claims 1 to 4, to the result of the wild type animal of identical species.

10. A preventive/remedy of autoimmune diseases or osteoporosis comprising a substance identified by the screening method of any of Claims 6 to 9.

11. A diagnostic agent of autoimmune diseases or osteoporosis comprising a partial or entire gene coding dendritic cell immune receptor (DCIR) protein.

12. The diagnostic agent of Claim 11 wherein the autoimmune disease is rheumatoid arthritis (RA), ankylosing spondylitis (AS), psoriatic arthritis (PsA), Sjogren syndrome (SS), or diffused idiopathic skeletal hyperostosis (DISH).

13. A kit to screen a preventive/remedy of autoimmune diseases or osteoporosis comprising a peptide having an amino acid sequence identical or substantially identical to dendritic cell immune receptor (DCIR) protein, a partial peptide, or a salt thereof, or a candidate substance.

14. A kit to diagnose autoimmune disease or osteoporosis comprising DNA having a partial or entire base sequence identical or substantially identical to gene coding dendritic cells immune receptor (DCIR) protein.

15. A remedy for infection including viral infection, bacterial infection, protozoan infection and fungal infection, comprising a peptide having an amino acid sequence identical or substantially identical to dendritic cell immune receptor (DCIR) protein, a partial peptide or a salt thereof.

16. An activating agent for cancer immunotherapy comprising a peptide having an amino acid sequence identical or substantially identical to dendritic cell immune receptor (DCIR) protein, a partial peptide or a salt thereof.


<110> Yoichiro Iwakura
GenoDive Pharma Inc.
<120> Dendric Cell Immunoreceptor gene knockout model animal
<130> PC0152GDP
<150> JP 2006-177952
<151> 2006-06-28
<150> JP 2007-030514
<151> 2007-02-09
<160> 17

<210> 1
<211> 2404
<212> DNA
<213> Mus musculus
<400> 1
ggcatttccc ttatctcgcc ctggtgattc tatgctgtgg tttcttgttc 50
tcatctcgtt tatcctagtg agacatgtct cttctttcat acaactgtgc 100
aatatgacaa cttatcacag tgattggttc tcatatacta tagagcctta 150
gagaaggaac aaggctctct tctgacggag gaagattttt tcttgatatg 200
gcttcagaaa tcacttatgc agaagtgaag ttcaagaatg aatccaactc 250
cttgcacacc tactcagaat ctcctgcagc tcccagagag aaacctatcc 300
gtgatctaag aaagcctggt tccccctcac tgcttcttac atccctgatg 350
ctacttctcc tgctgctggc aatcacattc ttagttgctt ttatcattta 400
ttttcaaaag tactctcaac ttcttgaaga aaaaaaagct gcaaaaaata 450
taatgcacaa tgaattgaac tgcacaaaaa gtgtttcacc catggaagac 500
aaagtctgga gctgttgccc aaaggattgg aggctatttg gttcccactg 550
ctacttggtt cccacagttt cttcatcagc atcttggaac aagagtgagg 600
agaactgctc ccgcatgggt gctcatctag tggtgatcca aagccaggaa 650
gagcaggatt tcatcactgg gatcttggac actcatgctg cttattttat 700
agggttgtgg gatacaggcc atcggcaatg gcaatgggtt gatcagacac 750
catatgaaga aagtatcaca ttctggcaca atggtgagcc cagcagtggc 800
aatgaaaaat gtgctacaat aatttaccgt tggaagactg gatggggctg 850
gaacgatatc tcttgcagtc ttaaacagaa gtcagtttgt cagatgaaga 900
aaataaactt atgaatcact cattcttcat gggcattcga ttcattgtta 950
tccaaccatt acacagacac ctgggaaatt ctacaggttc acagaattta 1000
agtgggcagc aaatggttat gcatacactg gcccacatat atccttgtgc 1050
atttacccac ctactctgtc ataaaatgaa ctttcattga gaattttcta 1100
tataccacag agtatacaga gtcccttatg gacacacatg gaactttttg 1150
ccatcttgtt tactcatgcc attgtatgat aggttctctt gacctatctg 1200
tttctgtttc tctgttgttt ttttaatgtc tttggattta ttgacattaa 1250
attgagaagt aaaattataa atatttaagt gtctggattg atacacacag 1300
atatgtacta tgaaatataa ttaaatattt actgtcaaat aattgtccaa 1350
agaaggttaa caacaccttc ctttacttaa tattattgct atattgcatg 1400
tgtatgtgtg acagaaagag aaagaggaga gagagagaga gagagagaga 1450
gagagagaga gagagagaga gagagagaga gagagagaga atgtgtgtgt 1500
gataagaata cttatggcca cttaaagaaa attctgataa ataggatgtt 1550
gctaacattt atcattttat tttaccatgg atttcatatt tatgtctctt 1600
ttatcagcag tttgttccct tcatcaacat gtcagagacc ccagtacccc 1650
accctttaca accatgtatt cttcactcaa attcctgaga ttatttgata 1700
aattattata ttgtaatatt ttacagatta tctcttcatt taatttctct 1750
aatgattcat gaaaagcagc attcccagct accaacatgc agtgattatt 1800
gttttgtgtg ttgattaaat gatgtttgag aacaagactg gtttatagtt 1850
tagacttaga taaatataga atgaaaaata aggcccaact tgctgatgta 1900
gctataatgc tcatctaaaa aatgtgaatt tatatactta atataaaata 1950

gaaatattaa gtattcttta ttagccatta tatgaaaacc atactaaaat 2000
gcctttcagt aagtaaaagg cagcatatta gacacaagat actctgacta 2050
aattggcata gagaagaaga atatagacaa ttgctttctt cttttcaaac 2100
attttctgta gcaagccaag ggtgacaaat ggaaacccag acaaaaccta 2150
actgagccct gagctagctc agacatattc atgtaaccag tttcataagt 2200
caatatgtga aataaaactg catcagcctc tgaggcagca ttcagaaaat 2250
tgaacattac tacttaagct aattttctct ttaggaaagg aaagcaagga 2300
tagtattctc taatctggaa ttggtcattt taagcattca ttttaccatg 2350
agtaaatttt acacgatttt actcaaaccc aataaaatat tctgctagcc 2400
cttc 2404

<210> 2
<211> 1291
<212> DNA
<213> Homo sapience
<400> 2
ctgtgattct cactatactg gtcctgagga aagggcttct gtgaactgcg 50
gtttttagtt tttattgtgg ttcttagttc tcatgagacc cctcttgagg 100
atatgtgcct atctggtgcc tctgctctcc actagttgag tgaaaggaag 150
gaggtaattt accaccatgt ttggttcctg tttataagat gttttaagaa 200
agatttgaaa cagattttct gaagaaagca gaagctctct tcccattatg 250
acttcggaaa tcacttatgc tgaagtgagg ttcaaaaatg aattcaagtc 300
ctcaggcatc aacacagcct cttctgcagc ttccaaggag aggactgccc 350
ctcacaaaag taataccgga ttccccaagc tgctttgtgc ctcactgttg 400
atatttttcc tgctattggc aatctcattc tttattgctt ttgtcatttt 450
ctttcaaaaa tattctcagc ttcttgaaaa aaagactaca aaagagctgg 500
ttcatacaac attggagtgt gtgaaaaaaa atatgcccgt ggaagagaca 550
gcctggagct gttgcccaaa gaattggaag tcatttagtt ccaactgcta 600
ctttatttct actgaatcag catcttggca agacagtgag aaggactgtg 650
ctagaatgga ggctcacctg ctggtgataa acactcaaga agagcaggat 700
ttcatcttcc agaatctgca agaagaatct gcttattttg tggggctctc 750
agatccagaa ggtcagcgac attggcaatg ggttgatcag acaccataca 800
atgaaagttc cacattctgg catccacgtg agcccagtga tcccaatgag 850
cgctgcgttg tgctaaattt tcgtaaatca cccaaaagat ggggctggaa 900
tgatgttaat tgtcttggtc ctcaaaggtc agtttgtgag atgatgaaga 950
tccacttatg aactgaacat tctccatgaa caggtggttg gattggtatc 1000
tgtcattgta gggatagata ataagctctt cttattcatg tgtaagggag 1050
gtccatagaa tttaggtggt ctgtcaacta ttctacttat gagagaattg 1100
gtctgtacat tgactgattc actttttcat aaagtgagca tttattgagc 1150
attttttcat gtgccagagc ctgtactgga ggcccccatt gtgcacacat 1200
ggagagaaca tgagtctctc ttaattttta tctggttgct aaagaattat 1250
ttaccaataa aattatatga tgtggtgtct caaaaaaaaa a 1291

<210> 3
<211> 12722
<212> DNA
<213> Mus musculus
<220>
<221> source
<222> 1..12722
<223> /chromosome="6"
Targeted sequence
<400> 3
   ctaaattgta agcgttaata ttttgttaaa attcgcgtta aatttttgtt 50
   aaatcagctc attttttaac caataggccg aaatcggcaa aatcccttat 100
   aaatcaaaag aatagaccga gatagggttg agtgttgttc cagtttggaa 150
   caagagtcca ctattaaaga acgtggactc caacgtcaaa gggcgaaaaa 200
   ccgtctatca gggcgatggc ccactacgtg aaccatcacc ctaatcaagt 250
   tttttggggt cgaggtgccg taaagcacta aatcggaacc ctaaagggag 300
   cccccgattt agagcttgac ggggaaagcc ggcgaacgtg gcgagaaagg 350
   aagggaagaa agcgaaagga gcgggcgcta gggcgctggc aagtgtagcg 400
   gtcacgctgc gcgtaaccac cacacccgcc gcgcttaatg cgccgctaca 450
   gggcgcgtcc cattcgccat tcaggctgcg caactgttgg gaagggcgat 500
   cggtgcgggc ctcttcgcta ttacgccagc tggcgaaagg gggatgtgct 550
   gcaaggcgat taagttgggt aacgccaggg ttttcccagt cacgacgttg 600
   taaaacgacg gccagtgagc gcgcgtaata cgactcacta tagggcgaat 650
   tggagctcca ccgcggtggc agaagattaa aacatattgc cagaattcgt 700
   ttgagatcag gcagagcaat tcagtcagaa gtcaagagaa gccagtttga 750
   atcagtcagg tttgagcgga gttaggccag aacagctgag ttgaaccagc 800
   cagtgagagt tcagaaagaa ctagaaaggc ttgggaatgg ctcttatgtc 850
   atcccctcat ccgaggaaat aaaaacattt acatagagca actattagta 900
   aactctaacc aacctccctg aatgaccaac aactactaac ccccatctat 950
   tggggaccta acatttatgc atcatctgaa gaattctcag aattccaaat 1000
   gtcacacaat tgcagaatct atttgcagct ggcaagatga tgccccttcc 1050
   agggcaagag gtaaatcata gttgttatgg tctgatttca acattcagaa 1100
   taactgtagc cattttgttc catgcctggg agctatttta tattgcttct 1150
   gtaacaagcc taccagccat tgacacagaa aagtgatttg cctcaaggac 1200
   atgctgacca cataccctgt tttgttcttt atgtttggac tgttctgcat 1250
   gaggtttgct aatcttaaga aattcctcaa agtctttctt aggactccag 1300
   aaatcaaagg tcaatatgaa ctattatgtc tgcaatggct tgagtcagag 1350
   ttgatcacca gacagccttt cctgcaccta catatgggtt cactgtggtt 1400
   ttgaagttga cactgaacaa tggtactaat aaaccaagaa gttatactta 1450
   caatactcat gctctgttat ctcaatgttc tgcaattccc ctgtttacca 1500
   cccatccacc actcacctca ctttactcag gaccaatcag cttaaaggct 1550
   agctgataat cctttggcta ctaaagtgta ctgctcccct tttgcctttt 1600
   aaacttttga acctgtttat atatatatgt gtatatatat atattctacc 1650
   ctgagaacag acaagaactg cacttatgcc ttctagtcat ttaatggtcc 1700
   tgattgttca gtattaaggt gtgcattcat tattcttgct taattgagat 1750
   caatgtttgc ttcattttag tggcaattac tggaacccaa caatggtcag 1800
   cggctgcaaa gcagcttgat atcccgacac ctgtgattaa aacaaaaatc 1850
   tagtcttata atatttctgt gtttttaaaa gaaaacaaaa ttcctcaatc 1900
   ctcactacat ttcccacttt ctgttttaag ccattaatta tgttgtgtgt 1950
   agcggggaat tataaacttc tgctcagttc taatctttat tgggtttgat 2000
   tgttattcac agcttttgga ttcctatgga aatataaaca aatccatctc 2050
   cctaatgcaa aactttcact ggtttccatt ctgatgttag cacatcctta 2100
   tacaggatga tttaattcca gaatatatat atatgtgtgt gtgtgtatat 2150
   atatacacac atatatatat tacctatatt gcaatgtttc ttggctgttt 2200
   tttctttctg aaatggttca ttcatttttt tttgctgtag taccttgagc 2250
   cccagaggac attttctgat ggtaagaaat taccctatct tttttggaca 2300
   tacattaaat tgggacagtg gcaatgcttg ccatatcacc tgcacacccc 2350
   taaaacttga ggtcttcttt ctgatttata ttgaaaagta ccattgctct 2400
   tagatatgct ttgatcatta acatactttc cacaattaat gcaccatgca 2450
   ttttgatatt cgagacctct cactataact tgacctatga tattagcatg 2500
   gtacacatta gaaccaatat tggtcacatc ccttatccac tcttccatgg 2550
   gtgctgtcat gcctttactg atctcataac ttgttttttg ggggggggtt 2600
   cgagacaggg tttctctgtg tagccctggc tgtcctggaa ctcactctgt 2650
   agatcaggct ggcctcgaac tcagaaatcc acctacctct gcctcccgat 2700
   tggtaggatt aaaggcatgc gccaccaacg cccggtgagc tcataacttt 2750
   tatattcaac attcatatct tcaaatgccc aggtctctca aaacctgcct 2800
   tgtttcaggg tctaatacag ccttgctctc agctgagatt aatctttgtt 2850
   acaacagcaa caacaacaac aacaacaaca acaacaacaa caacaacaac 2900
   aacaactacc agtgaaggct tctccagagc cttgtacaat cttagtaaat 2950
   aatgtggaca cttttccagg gctcctcaat tttgtcttaa ggtcttaaaa 3000
   ctgctaagca acatcattct atagaaacat catcaaacct gttcttatag 3050
   atcagagtat caaccttcac ccagcaactg atcttttatg atattcattc 3100
   ccctagcact aattcactgt tcaatatttg ctgtttcttc tgtctgccag 3150
   gttagtcatt gcaactgtgg tctaacctct aatactgctg ttaccaatcc 3200
   cttccagttt tggggaataa ctctactttg agaacctcag ttatttagaa 3250
   tttgttttgc ataagtttag tgcattctta tgaaattatg gcttctttaa 3300
   aattcattag agctattact tctatgggat accatcctat cttctcataa 3350
   ccttgttcct catcattaca ttagttgtat aacttttggg aaagctgtgg 3400
   tggtctataa ccctgggctg cccctccacc cactctggca gcacagttgg 3450
   ttaggaatta accctttccc tcaaaatggc ctgtttcatc agattgtact 3500
   cctgcttttt cagcttttct ctgacccatc caccctctgg cacaatttct 3550
   ttcctcattc ttcctgtggg aagtctccat tctcttgctc agtcactggg 3600
   cctgagcctt ttatgttctc ccttttcaat gatccttgct ctcttgtgct 3650
   tccatctcca cccacagttt ttccagttgc acagccaact ttacattcct 3700
   ttcagaaaca aaagaataga ttgacccctt ttgtttacac cgcctttttt 3750
   tttgtatttt gtgccccctc agttcgagtc acagtttttc taactcctca 3800
   gccagttttt caagtctttt ttgaaataaa atatatgaag aaggaaaaga 3850
   aaaaataaga gaaatccctc tgggagtaaa gtagagatac cccagtagta 3900
   gtagatgcaa taaacgtttt gctggcttct taaataccca tatccccccc 3950
   ccccaacatt tcttttatgg cacttgaaat caaaattttc attttatcct 4000
   taaattttga gctacaggct gagttgccat ttgttgttac attttgggtt 4050
   caccttttcc caccatttat ctctgctcct catcatttca cccccatata 4100
   actagatagg agaaagagga gagatagaaa gaagagaaag atagagatcc 4150
   ttgaatctaa tctcttgctt gtttcttctt tgagcatgac tactaataat 4200
   ctgaaaaccc ctccccctca caaccactaa ccaccaattc tgcctatcgg 4250
   ggctctagaa tttatatact ctctgaaaat ttcccaggat tccaaacatg 4300
   acacaattgc agaaactttc tgaaactgga ataagcacac cgccaccaga 4350
   tcatgaggtt aattatcgtc agctgctgtg ggaagtctga aacaagcaca 4400
   ggttgctgca cctgagattc agaatgaaaa tatattctta tatttctatg 4450
   ttttttaaaa gaagtaaaaa tttcaaaatt ctcaccacaa gcttccttgc 4500
   agagggtaga ccaggcatgg gtaaagagtg tgcatggtgg aaaactcact 4550
   gtgggacgtt actgtctatt aaggtaacga gaatatccat gcctggttgt 4600
   ggatggcctg acattgggtg ttagcagtca agtaggatga ggagtgtatc 4650
   tatgcacaga aggcagaaat ggtgtcactt tcagagaatc gtgaggtgag 4700
   aatgctgtct gcttggtggg atatctctgt gtcgctgctt ggtaccttcc 4750
   agggtgttga gagtttctgc ccatcaaggt gaccttggtg gtgtgctaaa 4800
   acccagggag gctgagtgga gtcttcccag agatcagcac caggaatgag 4850
   agcacatcca gcttagggga cataggcttg acctgaggtt tggagtccac 4900
   ccagaaagta caggcctcta agtgaagtgt gagcaacatt gtgggaaaga 4950
   gatgggagca tccaagagat tatcacttgg gagtttgaga tagagataat 5000
   gagcatagat ttgtttttac tcatagatgt agattagaaa tgccaaattt 5050
   tggaaataaa acgaaacctg tggctttaca ttaaaatggg ggtaatagtg 5100
   ttaacccatg gttttcagca tgtttggata taaaagcaaa agaaaaccag 5150
   taaatacaaa ctctctctct ctctctctgt gtgtcttatg catgcacatg 5200
   ttgtttgatt gttatgtgta gtgtaacatg gaagtagcta gaggagattc 5250
   aaagaaggga agtctttgga agaactgaag gctgagcatg tggctgtgag 5300
   ctggaagatg tctgtgggag tgactcaaat gattccatct ccagcagctc 5350
   aagtaagtgt cccctcttgt actctgtgat ccattttatt ctctcaacta 5400
   ttcccaacag tgcagtgaac atttaaagca tgagctgagg agggctggga 5450
   gtaagggaag agaggcacaa ggagaggaga gagtccaacc actgctacta 5500
   tccagagctg tttcaggagc tctgggcatg tccagtagca aagtgagtgg 5550
   agagtcccca tgcaggtagt tatgaatcta tggcaggagg cagagtgctc 5600
   agaggagcaa cttcagaaga aacagaatgt gaactttgcc ttccccacac 5650
   tggtggcagc tgagaaaaaa gtgggaatcc acacaatggt gagggctaat 5700
   ggtccactac aatgtggtat caaaattttc ctgttcagtc aaaatcatcg 5750
   ttgtgcctta agagtttgct tatgttgtct ggttttccag aaaccacatt 5800
   tgcatataga agtgagtgaa tttatgtgtg ctgcagagtg ttttacaaca 5850
   gggttctcta atagcatggt tcctctttct cctccttcct ctttctttcc 5900
   ttcccccttt gagttcccac tttgcagtac ttgcatatct tgctgagtgg 5950
   gtttgagggc tacaattctt attttcttat gttaagaggt tgcatttccc 6000
   ttatctcgcc ctggtgattc tatgctgtgg tttcttgttc tcatctcgtt 6050
   tatcctagtg agacatgtct cttctttcat acaactgtgc aatatgacaa 6100
   cttatcacag tgattggttc tcatatacta tagagcctta gagaaggaac 6150
   aaggctctct tctgacggag gaagattttt tcttgatggg ctgcagggaa 6200
   ttcgagcgac tcgagataac ttcgtataat gtatgctata cgaagttata 6250
   cgcgttcgga tttgagctcg ggtcgaccaa gaaaggcgga gtcttcggta 6300
   tctcgggtgg cgtaggggtc gtacggacga taacagaagg gttaggaggg 6350
   ggaacgacag gacggggtgg ggtggggggt cttatcttac tgtggatgag 6400
   tctgttacgc tacgttaaag gagtaaaata atcctttcct gtcaccctca 6450
   ccgtggaagg tcccagttcc ttccgtgccc cctccccgtt tgttgtctac 6500
   cgaccgttga tcttccgtgt cagctccgac tagtcgctcg agatctctta 6550
   actaggggag tcttcttgag cagttcttcc gctatcttcc gctacgcgac 6600
   gcttagccct cgccgctatg gcatttcgtg ctccttcgcc agtcgggtaa 6650
   gcggcggttc gagaagtcgt tatagtgccc atcggttgcg atacaggact 6700
   atcgccaggc ggtgtgggtc ggccggtgtc agctacttag gtcttttcgc 6750
   cggtaaaagg tggtactata agccgttcgt ccgtagcggt acccagtgct 6800
   gctctaggag cggcagcccg tacgcgcgga actcggaccg cttgtcaagc 6850
   cgaccgcgct cggggactac gagaagcagg tctagtagga ctagctgttc 6900
   tggccgaagg taggctcatg cacgagcgag ctacgctaca aagcgaacca 6950
   ccagcttacc cgtccatcgg cctagttcgc atacgtcggc ggcgtaacgt 7000
   agtcggtact acctatgaaa gagccgtcct cgttccactc tactgtcctc 7050
   taggacgggg ccgtgaagcg ggttatcgtc ggtcagggaa gggcgaagtc 7100
   actgttgcag ctcgtgtcga cgcgttcctt gcgggcagca ccggtcggtg 7150
   ctatcggcgc gacggagcag gacgtcaagt aagtcccgtg gcctgtccag 7200
   ccagaactgt ttttcttggc ccgcggggac gcgactgtcg gccttgtgcc 7250
   gccgtagtct cgtcggctaa cagacaacac gggtcagtat cggcttatcg 7300
   gagaggtggg ttcgccggcc tcttggacgc acgttaggta gaacaagtta 7350
   ccggctaggg tataaccgac gtccagcttt ccgggcctct actccttctc 7400
   ctcttgtcgc gccgtctgca cgcgaaaact tcgcacgctc ttacggcccg 7450
   gaggcctcct ggaagcccgc gggcggggcg gggactcggg cggggactcg 7500
   ggcgggggcc tgggtgggga agggtcggag actcgggtct ttcgcttcct 7550
   cgtttcgacg ataaccggcg acggggtttc cggatgggcg aaggtaacga 7600
   gtcgccacga caggtagacg tgctctgatc actctgcacg atgaaggtaa 7650
   acagtgcagg acgtgctgcg ctcgacgccc cgccccccct tgaaggactg 7700
   atcccctcct catcttccac cgcgcttccc cggtggtttc ttgcctcggc 7750
   caaccgcgga tggccaccta caccttacac acgctccggt ctccggtgaa 7800
   cacatcgcgg ttcacgggtc gccccgacga tttcgcgtac gaggtctgac 7850
   ggaacccttt tcgcggaggg gatgggccat cttaaggaaa tccgactcga 7900
   gataacttcg tataatgtat gctatacgaa gttatacgcg ttcgctcggt 7950
   acccatcaag cttatctgca cacatcaatg gaccatgtct gccttagatg 8000
   aagagaggtt ccactctagc agttggcatt taatggaaat atgaagaaga 8050
   aatattcttc atggaaaatt aatacaaggg attctattgt gacttcccct 8100
   ttgtccttaa atttaattat tggggaaaaa tggagtccac tctcagtata 8150
   gaaactaaat cacactcgtg agtgtctgtg tggggctgtc tttattagac 8200
   tgggaggtca aatctacatt ttctcattgg cagcaaggtg tcaaactgta 8250
   tgtgtatagg gctccttcat ctaatgtgag cttgtagaca agaaagagct 8300
   aggaagaaat tgtccaagaa ggagttcttc agctaattcc tagctgcttc 8350
   tgtnnnnnnn nnnnnnnnnn nnnnnnnnnn nnnnnnnnnn nnnnnnnnnn 8400
   nnnnnnnnnn nnnnnnnnnn nnnnnnnnnn nnnnnnnnnn nnnnnntgta 8450
   ttacagatga tgtatcagac caagtcccag gaagaacaca gaactctgtt 8500
   ctcaagaaat gtagaggaaa gttaatgaat cagtgtcagc taaatataac 8550
   ggcaatctct tattatccca gctacttagg atgttagata gtaggacagt 8600
   aagtgtggag ctagcctggg ctatgtaaaa agactatatc tgatgaaaat 8650
   aaactggggt ctgcaacatt gtttgggaag tgttgtattg tgatagcttt 8700
   cctaaaatag gtgacaccta gaactgtggg taccaagaat gtcacagagg 8750
   gatgaaactg aagcacgagt ggctgaaata cacagtgcta agtgaagaag 8800
   aaatgagatt tccctaggta gacacaagca ggatgtagaa catgtgctgt 8850
   gcacccatac aaaaccattt atttctctta aaatgtgttc tttaatcaag 8900
   tattgttttc tacttaataa atccatgtca atgtaatgag ctaatacata 8950
   tacttattta tctcatgctt ctatggtttg gcaatttagg ctgatctcca 9000
   ctgagtggct cctcttgtcc tagttgagct taggcatgtg tatgtgtcca 9050
   ggcatctcag actctgagga agaggaggcc agctggaatg acgaacagtg 9100
   tggctcaact ttatatgagt tcttaggagg gcctgggctc atttacatgg 9150
   cagcttggag atttccaaga ctgaaggtgg caatgagcca aggctctcaa 9200
   aaaatttaac tggagagtac atcagagatc atttttcaca ttctcttgtt 9250
   cagctcttgc agaaaacttt actttttttt ttttaaattt aaattgggac 9300
   agttgcaaaa tcccattgca atagtatgtg gacatagaag ttaaaaattg 9350
   tggtcatttt tttttttttt gcaaaatatc aatgccatgg agaactgctg 9400
   taccatttca aagagagaat tggtatagcc acatttaggt tatagatggt 9450
   gctttttaca ggaatctgaa aatagaatct gtagagcaaa gagagaaatg 9500
   agagaaataa tttataacct gataatgaga taagtgtcag gaaaacatga 9550
   gatgaggcag ctttgagatt taaagagtat aggaccaaga aaatagatgt 9600
   ttgatgatta ttgagatcta ggaattaaga ggtccaagaa ataaaatgtg 9650
   attttaatag tttgatgatg taaactcagc aatcatgaaa ttattcaaag 9700
   aggtgtagaa tatataagcc atatgtttaa gttggtagat gttgacttga 9750
   gttttagaca tactgagttg aacctaatat gattgcattt agtgaccata 9800
   aaatcttatg caaaagggag cagacaagca ataccatagc aatacatgac 9850
   cttactcagg aagaatttat atgagaagaa atcataatga cagggccgta 9900
   ggaagtgtta aattgaaggg gtggatggaa ggagaagaga ttattaaaga 9950
   caataaaaag tgattgtcaa aaggtgaggc aaaaccaaga caggcagcgt 10000
   ctaaagacct aagggttaag agaactaagc ttacatccaa taacaagatg 10050
   gctaatgagt gccctgggat attactgagt atacatttca aaactggtgt 10100
   ttgagaaatg gaggaagcat aagtgatggc ataatgagga caacaacata 10150
   ggcaggaaag tgaggaaatc tagttctaga gggggaggcc agttagtttt 10200
   aataggacag aatgggagtg tggaacatgg aggaataagt cagtctaaat 10250
   gaagaaagtt taaggaagaa agcaatgccc tgcagatact tgtggttttt 10300
   tttttttttc tgatttcaca ttctaaaata acatataact actcagaatt 10350
   acttaactcc ttttcctccc aatgcaaagg tagttgacat ccttaaatat 10400
   gactatttat ctctgattca tctcctggaa gaattaacat tttcatctta 10450
   cattcagctt tgaaggatat ggttcactcc ctttagacga taccgtcgac 10500
   ctcgaggggg ggcccggtac ccagcttttg ttccctttag tgagggttaa 10550
   ttgcgcgctt ggcgtaatca tggtcatagc tgtttcctgt gtgaaattgt 10600
   tatccgctca caattccaca caacatacga gccggaagca taaagtgtaa 10650
   agcctggggt gcctaatgag tgagctaact cacattaatt gcgttgcgct 10700
   cactgcccgc tttccagtcg ggaaacctgt cgtgccagct gcattaatga 10750
   atcggccaac gcgcggggag aggcggtttg cgtattgggc gctcttccgc 10800
   ttcctcgctc actgactcgc tgcgctcggt cgttcggctg cggcgagcgg 10850
   tatcagctca ctcaaaggcg gtaatacggt tatccacaga atcaggggat 10900
   aacgcaggaa agaacatgtg agcaaaaggc cagcaaaagg ccaggaaccg 10950
   taaaaaggcc gcgttgctgg cgtttttcca taggctccgc ccccctgacg 11000
   agcatcacaa aaatcgacgc tcaagtcaga ggtggcgaaa cccgacagga 11050
   ctataaagat accaggcgtt tccccctgga agctccctcg tgcgctctcc 11100
   tgttccgacc ctgccgctta ccggatacct gtccgccttt ctcccttcgg 11150
   gaagcgtggc gctttctcat agctcacgct gtaggtatct cagttcggtg 11200
   taggtcgttc gctccaagct gggctgtgtg cacgaacccc ccgttcagcc 11250
   cgaccgctgc gccttatccg gtaactatcg tcttgagtcc aacccggtaa 11300
   gacacgactt atcgccactg gcagcagcca ctggtaacag gattagcaga 11350
   gcgaggtatg taggcggtgc tacagagttc ttgaagtggt ggcctaacta 11400
   cggctacact agaaggacag tatttggtat ctgcgctctg ctgaagccag 11450
   ttaccttcgg aaaaagagtt ggtagctctt gatccggcaa acaaaccacc 11500
   gctggtagcg gtggtttttt tgtttgcaag cagcagatta cgcgcagaaa 11550
   aaaaggatct caagaagatc ctttgatctt ttctacgggg tctgacgctc 11600
   agtggaacga aaactcacgt taagggattt tggtcatgag attatcaaaa 11650
   aggatcttca cctagatcct tttaaattaa aaatgaagtt ttaaatcaat 11700
   ctaaagtata tatgagtaaa cttggtctga cagttaccaa tgcttaatca 11750
   gtgaggcacc tatctcagcg atctgtctat ttcgttcatc catagttgcc 11800
   tgactccccg tcgtgtagat aactacgata cgggagggct taccatctgg 11850
   ccccagtgct gcaatgatac cgcgagaccc acgctcaccg gctccagatt 11900
   tatcagcaat aaaccagcca gccggaaggg ccgagcgcag aagtggtcct 11950
   gcaactttat ccgcctccat ccagtctatt aattgttgcc gggaagctag 12000
   agtaagtagt tcgccagtta atagtttgcg caacgttgtt gccattgcta 12050
   caggcatcgt ggtgtcacgc tcgtcgtttg gtatggcttc attcagctcc 12100
   ggttcccaac gatcaaggcg agttacatga tcccccatgt tgtgcaaaaa 12150
   agcggttagc tccttcggtc ctccgatcgt tgtcagaagt aagttggccg 12200
   cagtgttatc actcatggtt atggcagcac tgcataattc tcttactgtc 12250
   atgccatccg taagatgctt ttctgtgact ggtgagtact caaccaagtc 12300
   attctgagaa tagtgtatgc ggcgaccgag ttgctcttgc ccggcgtcaa 12350
   tacgggataa taccgcgcca catagcagaa ctttaaaagt gctcatcatt 12400
   ggaaaacgtt cttcggggcg aaaactctca aggatcttac cgctgttgag 12450
   atccagttcg atgtaaccca ctcgtgcacc caactgatct tcagcatctt 12500
   ttactttcac cagcgtttct gggtgagcaa aaacaggaag gcaaaatgcc 12550
   gcaaaaaagg gaataagggc gacacggaaa tgttgaatac tcatactctt 12600
   cctttttcaa tattattgaa gcatttatca gggttattgt ctcatgagcg 12650
   gatacatatt tgaatgtatt tagaaaaata aacaaatagg ggttccgcgc 12700
   acatttcccc gaaaagtgcc ac 12722

<210> 4
<211> 22
<212> DNA
<213> Artificial sequence
<220>
<223> PCR primer
<400> 4
catttccctt atctcgccct gg 22

<210> 5
<211> 22
<212> DNA
<213> Artificial sequence
<220>
<223> PCR primer

<400> 5
gcagcatgaa tgtccaagat cc      22

<210> 6
<211> 38
<212> DNA
<213> Artificial sequence
<220>
<223> PCR primer
<400> 6
gattaaaagc ggccgccaga attcgtttga gatcaggc      38

<210> 7
<211> 29
<212> DNA
<213> Artificial sequence
<220>
<223> PCR primer
<400> 7
ctggatccgt cagaagagag ccttgttcc      29

<210> 8
<211> 29
<212> DNA
<213> Artificial sequence
<220>
<223> PCR primer
<400> 8
ccatcgatga agagaggttc cactctagc      29

<210> 9
<211> 29
<212> DNA
<213> Artificial sequence
<220>
<223> PCR primer
<400> 9
ttatcgatgt caactacctt tgcattggg      29

<210> 10
<211> 22
<212> DNA
<213> Artificial sequence
<220>
<223> primer for amplification
<400> 10
taacactgag ggaagatgct ac      22

<210> 11
<211> 22
<212> DNA
<213> Artificial sequence
<220>
<223> primer for amplification

<400> 11
tctcattctc actctcactc tc        22

<210> 12
<211> 18
<212> DNA
<213> Artificial sequence
<220>
<223> primer for amplification
<400> 12
agccatgata acagaccc        18

<210> 13
<211> 19
<212> DNA
<213> Artificial sequence
<220>
<223> primer for amplification
<400> 13
tgatatgggg tctggtacg        19

<210> 14
<211> 24
<212> DNA
<213> Artificial sequence
<220>
<223> PCR primer
<400> 14
aagtgtcccc tcttgtactc tgtg        24

<210> 15
<211> 25
<212> DNA
<213> Artificial sequence
<220>
<223> primer for amplification
<400> 15
caaaattctg tcaagcgtag agggg        25

<210> 16
<211> 26
<212> DNA
<213> Artificial sequence
<220>
<223> primer for amplification
<400> 16
cattatacga agttatctcg agtcgc        26

<210> 17
<211> 12818
<212> DNA
<213> Mus musculus
<220>
<221> source

<222> 1..12818
<223> /chromosome="6"
      Targeted sequence
<400> 17
ctaaattgta agcgttaata ttttgttaaa attcgcgtta aatttttgtt      50
aaatcagctc attttttaac caataggccg aaatcggcaa aatcccttat      100
aaatcaaaag aatagaccga gatagggttg agtgttgttc cagtttggaa      150
caagagtcca ctattaaaga acgtggactc caacgtcaaa gggcgaaaaa      200
ccgtctatca gggcgatggc ccactacgtg aaccatcacc ctaatcaagt      250
tttttggggt cgaggtgccg taaagcacta aatcggaacc ctaaagggag      300
cccccgattt agagcttgac ggggaaagcc ggcgaacgtg gcgagaaagg      350
aagggaagaa agcgaaagga gcgggcgcta gggcgctggc aagtgtagcg      400
gtcacgctgc gcgtaaccac cacacccgcc gcgcttaatg cgccgctaca      450
gggcgcgtcc cattcgccat tcaggctgcg caactgttgg gaagggcgat      500
cggtgcgggc ctcttcgcta ttacgccagc tggcgaaagg gggatgtgct      550
gcaaggcgat taagttgggt aacgccaggg ttttcccagt cacgacgttg      600
taaaacgacg gccagtgagc gcgcgtaata cgactcacta tagggcgaat      650
tggagctcca ccgcggtggc agaagattaa aacatattgc cagaattcgt      700
ttgagatcag gcagagcaat tcagtcagaa gtcaagagaa gccagtttga      750
atcagtcagg tttgagcgga gttaggccag aacagctgag ttgaaccagc      800
cagtgagagt tcagaaagaa ctagaaaggc ttgggaatgg ctcttatgtc      850
atcccctcat ccgaggaaat aaaaacattt acatagagca actattagta      900
aactctaacc aacctccctg aatgaccaac aactactaac ccccatctat      950
tggggaccta acatttatgc atcatctgaa gaattctcag aattccaaat      1000
gtcacacaat tgcagaatct atttgcagct ggcaagatga tgccccttcc      1050
agggcaagag gtaaatcata gttgttatgg tctgatttca acattcagaa      1100
taactgtagc cattttgttc catgcctggg agctatttta tattgcttct      1150
gtaacaagcc taccagccat tgacacagaa aagtgatttg cctcaaggac      1200
atgctgacca cataccctgt tttgttcttt atgtttggac tgttctgcat      1250
gaggtttgct aatcttaaga aattcctcaa agtctttctt aggactccag      1300
aaatcaaagg tcaatatgaa ctattatgtc tgcaatggct tgagtcagag      1350
ttgatcacca gacagccttt cctgcaccta catatgggtt cactgtggtt      1400
ttgaagttga cactgaacaa tggtactaat aaaccaagaa gttatactta      1450
caatactcat gctctgttat ctcaatgttc tgcaattccc ctgtttacca      1500
cccatccacc actcacctca ctttactcag gaccaatcag cttaaaggct      1550
agctgataat cctttggcta ctaaagtgta ctgctcccct tttgcctttt      1600
aaacttttga acctgtttat atatatatgt gtatatatat atattctacc      1650
ctgagaacag acaagaactg cacttatgcc ttctagtcat ttaatggtcc      1700
tgattgttca gtattaaggt gtgcattcat tattcttgct taattgagat      1750
caatgtttgc ttcattttag tggcaattac tggaacccaa caatggtcag      1800
cggctgcaaa gcagcttgat atcccgacac ctgtgattaa aacaaaaatc      1850
tagtcttata atatttctgt gtttttaaaa gaaaacaaaa ttcctcaatc      1900
ctcactacat ttcccacttt ctgttttaag ccattaatta tgttgtgtgt      1950
agcggggaat tataaacttc tgctcagttc taatctttat tgggtttgat      2000
tgttattcac agcttttgga ttcctatgga aatataaaca aatccatctc      2050
cctaatgcaa aactttcact ggtttccatt ctgatgttag cacatcctta      2100
tacaggatga tttaattcca gaatatatat atatgtgtgt gtgtgtatat      2150
atatacacac atatatatat tacctatatt gcaatgtttc ttggctgttt      2200
tttctttctg aaatggttca ttcatttttt tttgctgtag taccttgagc      2250
cccagaggac attttctgat ggtaagaaat taccctatct tttttggaca      2300
tacattaaat tgggacagtg gcaatgcttg ccatatcacc tgcacacccc      2350
taaaacttga ggtcttcttt ctgatttata ttgaaaagta ccattgctct      2400
tagatatgct ttgatcatta acatactttc cacaattaat gcaccatgca      2450
ttttgatatt cgagacctct cactataact tgacctatga tattagcatg      2500

gtacacatta gaaccaatat tggtcacatc ccttatccac tcttccatgg 2550
gtgctgtcat gcctttactg atctcataac ttgttttttg ggggggggtt 2600
cgagacaggg tttctctgtg tagccctggc tgtcctggaa ctcactctgt 2650
agatcaggct ggcctcgaac tcagaaatcc acctacctct gcctcccgat 2700
tggtaggatt aaaggcatgc gccaccaacg cccggtgagc tcataacttt 2750
tatattcaac attcatatct tcaaatgccc aggtctctca aaacctgcct 2800
tgtttcaggg tctaatacag ccttgctctc agctgagatt aatctttgtt 2850
acaacagcaa caacaacaac aacaacaaca acaacaacaa caacaacaac 2900
aacaactacc agtgaaggct tctccagagc cttgtacaat cttagtaaat 2950
aatgtggaca cttttccagg gctcctcaat tttgtcttaa ggtcttaaaa 3000
ctgctaagca acatcattct atagaaacat catcaaacct gttcttatag 3050
atcagagtat caaccttcac ccagcaactg atcttttatg atattcattc 3100
ccctagcact aattcactgt tcaatatttg ctgtttcttc tgtctgccag 3150
gttagtcatt gcaactgtgg tctaacctct aatactgctg ttaccaatcc 3200
cttccagttt tggggaataa ctctactttg agaacctcag ttatttagaa 3250
tttgttttgc ataagtttag tgcattctta tgaaattatg gcttctttaa 3300
aattcattag agctattact tctatgggat accatcctat cttctcataa 3350
ccttgttcct catcattaca ttagttgtat aacttttggg aaagctgtgg 3400
tggtctataa ccctgggctg cccctccacc cactctggca gcacagttgg 3450
ttaggaatta accctttccc tcaaaatggc ctgtttcatc agattgtact 3500
cctgcttttt cagcttttct ctgacccatc caccctctgg cacaatttct 3550
ttcctcattc ttcctgtggg aagtctccat tctcttgctc agtcactggg 3600
cctgagcctt ttatgttctc ccttttcaat gatccttgct ctcttgtgct 3650
tccatctcca cccacagttt ttccagttgc acagccaact ttacattcct 3700
ttcagaaaca aaagaataga ttgacccctt ttgtttacac cgcctttttt 3750
tttgtatttt gtgccccctc agttcgagtc acagtttttc taactcctca 3800
gccagttttt caagtctttt ttgaaataaa atatatgaag aaggaaaaga 3850
aaaaataaga gaaatccctc tgggagtaaa gtagagatac cccagtagta 3900
gtagatgcaa taaacgtttt gctggcttct taaataccca tatccccccc 3950
ccccaacatt tcttttatgg cacttgaaat caaaattttc attttatcct 4000
taaattttga gctacaggct gagttgccat ttgttgttac attttgggtt 4050
caccttttcc caccatttat ctctgctcct catcatttca cccccatata 4100
actagatagg agaaagagga gagatagaaa gaagagaaag atagagatcc 4150
ttgaatctaa tctcttgctt gtttcttctt tgagcatgac tactaataat 4200
ctgaaaaccc ctccccctca caaccactaa ccaccaattc tgcctatcgg 4250
ggctctagaa tttatatact ctctgaaaat ttcccaggat tccaaacatg 4300
acacaattgc agaaactttc tgaaactgga ataagcacac cgccaccaga 4350
tcatgaggtt aattatcgtc agctgctgtg ggaagtctga aacaagcaca 4400
ggttgctgca cctgagattc agaatgaaaa tatattctta tatttctatg 4450
ttttttaaaa gaagtaaaaa tttcaaaatt ctcaccacaa gcttccttgc 4500
agagggtaga ccaggcatgg gtaaagagtg tgcatggtgg aaaactcact 4550
gtgggacgtt actgtctatt aaggtaacga gaatatccat gcctggttgt 4600
ggatggcctg acattgggtg ttagcagtca agtaggatga ggagtgtatc 4650
tatgcacaga aggcagaaat ggtgtcactt tcagagaatc gtgaggtgag 4700
aatgctgtct gcttggtggg atatctctgt gtcgctgctt ggtaccttcc 4750
agggtgttga gagtttctgc ccatcaaggt gaccttggtg gtgtgctaaa 4800
acccagggag gctgagtgga gtcttcccag agatcagcac caggaatgag 4850
agcacatcca gcttagggga cataggcttg acctgaggtt tggagtccac 4900
ccagaaagta caggcctcta agtgaagtgt gagcaacatt gtgggaaaga 4950
gatgggagca tccaagagat tatcacttgg gagtttgaga tagagataat 5000
gagcatagat ttgtttttac tcatagatgt agattagaaa tgccaaattt 5050
tggaaataaa acgaaacctg tggctttaca ttaaaatggg ggtaatagtg 5100
ttaacccatg gttttcagca tgtttggata taaaagcaaa agaaaaccag 5150
taaatacaaa ctctctctct ctctctctgt gtgtcttatg catgcacatg 5200

ttgtttgatt gttatgtgta gtgtaacatg gaagtagcta gaggagattc 5250
aaagaaggga agtctttgga agaactgaag gctgagcatg tggctgtgag 5300
ctggaagatg tctgtgggag tgactcaaat gattccatct ccagcagctc 5350
aagtaagtgt cccctcttgt actctgtgat ccattttatt ctctcaacta 5400
ttcccaacag tgcagtgaac atttaaagca tgagctgagg agggctggga 5450
gtaagggaag agaggcacaa ggagaggaga gagtccaacc actgctacta 5500
tccagagctg tttcaggagc tctgggcatg tccagtagca aagtgagtgg 5550
agagtcccca tgcaggtagt tatgaatcta tggcaggagg cagagtgctc 5600
agaggagcaa cttcagaaga aacagaatgt gaactttgcc ttccccacac 5650
tggtggcagc tgagaaaaaa gtgggaatcc acacaatggt gagggctaat 5700
ggtccactac aatgtggtat caaaattttc ctgttcagtc aaaatcatcg 5750
ttgtgcctta agagtttgct tatgttgtct ggttttccag aaaccacatt 5800
tgcatataga agtgagtgaa tttatgtgtg ctgcagagtg ttttacaaca 5850
gggttctcta atagcatggt tcctctttct cctccttcct ctttctttcc 5900
ttcccccttt gagttcccac tttgcagtac ttgcatatct tgctgagtgg 5950
gtttgagggc tacaattctt attttcttat gttaagaggt tgcatttccc 6000
ttatctcgcc ctggtgattc tatgctgtgg tttcttgttc tcatctcgtt 6050
tatcctagtg agacatgtct cttctttcat acaactgtgc aatatgacaa 6100
cttatcacag tgattggttc tcatatacta tagagcctta gagaaggaac 6150
aaggctctct tctgacggag gaagattttt tcttgatggg ctgcagggaa 6200
ttcgagcgac tcgagataac ttcgtataat gtatgctata cgaagttata 6250
cgcgttcgga tttgagctcg ggtcgaccaa gaaaggcgga gtcttcggta 6300
tctcgggtgg cgtaggggtc gtacggacga taacagaagg gttaggaggg 6350
ggaacgacag gacggggtgg ggtggggggt cttatcttac tgtggatgag 6400
tctgttacgc tacgttaaag gagtaaaata atcctttcct gtcaccctca 6450
ccgtggaagg tcccagttcc ttccgtgccc cctccccgtt tgttgtctac 6500
cgaccgttga tcttccgtgt cagctccgac tagtcgctcg agatctctta 6550
actaggggag tcttcttgag cagttcttcc gctatcttcc gctacgcgac 6600
gcttagccct cgccgctatg gcatttcgtg ctccttcgcc agtcgggtaa 6650
gcggcggttc gagaagtcgt tatagtgccc atcggttgcg atacaggact 6700
atcgccaggc ggtgtgggtc ggccggtgtc agctacttag gtcttttcgc 6750
cggtaaaagg tggtactata agccgttcgt ccgtagcggt acccagtgct 6800
gctctaggag cggcagcccg tacgcgcgga actcggaccg cttgtcaagc 6850
cgaccgcgct cggggactac gagaagcagg tctagtagga ctagctgttc 6900
tggccgaagg taggctcatg cacgagcgag ctacgctaca aagcgaacca 6950
ccagcttacc cgtccatcgg cctagttcgc atacgtcggc ggcgtaacgt 7000
agtcggtact acctatgaaa gagccgtcct cgttccactc tactgtcctc 7050
taggacgggg ccgtgaagcg ggttatcgtc ggtcagggaa gggcgaagtc 7100
actgttgcag ctcgtgtcga cgcgttcctt gcgggcagca ccggtcggtg 7150
ctatcggcgc gacggagcag gacgtcaagt aagtcccgtg gcctgtccag 7200
ccagaactgt ttttcttggc ccgcggggac gcgactgtcg gccttgtgcc 7250
gccgtagtct cgtcggctaa cagacaacac gggtcagtat cggcttatcg 7300
gagaggtggg ttcgccggcc tcttggacgc acgttaggta gaacaagtta 7350
ccggctaggg tataaccgac gtccagcttt ccgggcctct actccttctc 7400
ctcttgtcgc gccgtctgca cgcgaaaact tcgcacgctc ttacggcccg 7450
gaggcctcct ggaagcccgc gggcggggcg gggactcggg cggggactcg 7500
ggcgggggcc tgggtgggga agggtcggag actcgggtct ttcgcttcct 7550
cgtttcgacg ataaccggcg acggggtttc cggatgggcg aaggtaacga 7600
gtcgccacga caggtagacg tgctctgatc actctgcacg atgaaggtaa 7650
acagtgcagg acgtgctgcg ctcgacgccc cgccccccct tgaaggactg 7700
atcccctcct catcttccac cgcgcttccc cggtggtttc ttgcctcggc 7750
caaccgcgga tggccaccta caccttacac acgctccggt ctccggtgaa 7800
cacatcgcgg ttcacgggtc gccccgacga tttcgcgtac gaggtctgac 7850
ggaacccttt tcgcggaggg gatgggccat cttaaggaaa tccgactcga 7900

gataacttcg tataatgtat gctatacgaa gttatacgcg ttcgctcggt 7950
acccatcaag cttatctgca cacatcaatg gaccatgtct gccttagatg 8000
aagagaggtt ccactctagc agttggcatt taatggaaat atgaagaaga 8050
aatattcttc atggaaaatt aatacaaggg attctattgt gacttcccct 8100
ttgtccttaa atttaattat tggggaaaaa tggagtccac tctcagtata 8150
gaaactaaat cacactcgtg agtgtctgtg tggggctgtc tttattagac 8200
tgggaggtca aatctacatt ttctcattgg cagcaaggtg tcaaactgta 8250
tgtgtatagg gctccttcat ctaatgtgag cttgtagaca agaaagagct 8300
aggaagaaat tgtccaagaa ggagttcttc agctaattcc tagctgcttc 8350
tgtcttgtcc agtattatga gaaatctaaa agcaacctga gcaaatgggt 8400
ggatttctgt tcatcaaaga tttgagaatg tatgaaaaat ttaaaaaaat 8450
gcatgaaaag tcacatattt aggatgcttt actcatttat ttgcacactc 8500
agaataaagt aatagtaata atatagtaat agcacacatt aatgtattac 8550
agatgatgta tcagaccaag tcccaggaag aacacagaac tctgttctca 8600
agaaatgtag aggaaagtta atgaatcagt gtcagctaaa tataacggca 8650
atctcttatt atcccagcta cttaggatgt tagatagtag gacagtaagt 8700
gtggagctag cctgggctat gtaaaaagac tatatctgat gaaaataaac 8750
tggggtctgc aacattgttt gggaagtgtt gtattgtgat agctttccta 8800
aaataggtga cacctagaac tgtgggtacc aagaatgtca cagagggatg 8850
aaactgaagc acgagtggct gaaatacaca gtgctaagtg aagaagaaat 8900
gagatttccc taggtagaca caagcaggat gtagaacatg tgctgtgcac 8950
ccatacaaaa ccatttattt ctcttaaaat gtgttcttta atcaagtatt 9000
gttttctact taataaatcc atgtcaatgt aatgagctaa tacatatact 9050
tatttatctc atgcttctat ggtttggcaa tttaggctga tctccactga 9100
gtggctcctc ttgtcctagt tgagcttagg catgtgtatg tgtccaggca 9150
tctcagactc tgaggaagag gaggccagct ggaatgacga acagtgtggc 9200
tcaactttat atgagttctt aggagggcct gggctcattt acatggcagc 9250
ttggagattt ccaagactga aggtggcaat gagccaaggc tctcaaaaaa 9300
tttaactgga gagtacatca gagatcattt ttcacattct cttgttcagc 9350
tcttgcagaa aactttactt tttttttttt aaatttaaat tgggacagtt 9400
gcaaaatccc attgcaatag tatgtggaca tagaagttaa aaattgtggt 9450
catttttttt ttttttgcaa aatatcaatg ccatggagaa ctgctgtacc 9500
atttcaaaga gagaattggt atagccacat ttaggttata gatggtgctt 9550
tttacaggaa tctgaaaata gaatctgtag agcaaagaga gaaatgagag 9600
aaataattta taacctgata atgagataag tgtcaggaaa acatgagatg 9650
aggcagcttt gagatttaaa gagtatagga ccaagaaaat agatgtttga 9700
tgattattga gatctaggaa ttaagaggtc caagaaataa aatgtgattt 9750
taatagtttg atgatgtaaa ctcagcaatc atgaaattat tcaaagaggt 9800
gtagaatata taagccatat gtttaagttg gtagatgttg acttgagttt 9850
tagacatact gagttgaacc taatatgatt gcatttagtg accataaaat 9900
cttatgcaaa agggagcaga caagcaatac catagcaata catgacctta 9950
ctcaggaaga atttatatga gaagaaatca taatgacagg gccgtaggaa 10000
gtgttaaatt gaaggggtgg atggaaggag aagagattat taaagacaat 10050
aaaaagtgat tgtcaaaagg tgaggcaaaa ccaagacagg cagcgtctaa 10100
agacctaagg gttaagagaa ctaagcttac atccaataac aagatggcta 10150
atgagtgccc tgggatatta ctgagtatac atttcaaaac tggtgtttga 10200
gaaatggagg aagcataagt gatggcataa tgaggacaac aacataggca 10250
ggaaagtgag gaaatctagt tctagagggg gaggccagtt agttttaata 10300
ggacagaatg ggagtgtgga acatggagga ataagtcagt ctaaatgaag 10350
aaagtttaag gaagaaagca atgccctgca gatacttgtg gttttttttt 10400
tttttctgat ttcacattct aaaataacat ataactactc agaattactt 10450
aactcctttt cctcccaatg caaaggtagt tgacatcctt aaatatgact 10500
atttatctct gattcatctc ctggaagaat taacattttc atcttacatt 10550
cagctttgaa ggatatggtt cactcccttt agacgatacc gtcgacctcg 10600

agggggggcc cggtacccag cttttgttcc ctttagtgag ggttaattgc 10650
gcgcttggcg taatcatggt catagctgtt tcctgtgtga aattgttatc 10700
cgctcacaat tccacacaac atacgagccg gaagcataaa gtgtaaagcc 10750
tggggtgcct aatgagtgag ctaactcaca ttaattgcgt tgcgctcact 10800
gcccgctttc cagtcgggaa acctgtcgtg ccagctgcat taatgaatcg 10850
gccaacgcgc ggggagaggc ggtttgcgta ttgggcgctc ttccgcttcc 10900
tcgctcactg actcgctgcg ctcggtcgtt cggctgcggc gagcggtatc 10950
agctcactca aaggcggtaa tacggttatc cacagaatca ggggataacg 11000
caggaaagaa catgtgagca aaaggccagc aaaaggccag gaaccgtaaa 11050
aaggccgcgt tgctggcgtt tttccatagg ctccgccccc ctgacgagca 11100
tcacaaaaat cgacgctcaa gtcagaggtg gcgaaacccg acaggactat 11150
aaagatacca ggcgtttccc cctggaagct ccctcgtgcg ctctcctgtt 11200
ccgaccctgc cgcttaccgg atacctgtcc gcctttctcc cttcgggaag 11250
cgtggcgctt tctcatagct cacgctgtag gtatctcagt tcggtgtagg 11300
tcgttcgctc caagctgggc tgtgtgcacg aaccccccgt tcagcccgac 11350
cgctgcgcct tatccggtaa ctatcgtctt gagtccaacc cggtaagaca 11400
cgacttatcg ccactggcag cagccactgg taacaggatt agcagagcga 11450
ggtatgtagg cggtgctaca gagttcttga agtggtggcc taactacggc 11500
tacactagaa ggacagtatt tggtatctgc gctctgctga agccagttac 11550
cttcggaaaa agagttggta gctcttgatc cggcaaacaa accaccgctg 11600
gtagcggtgg tttttttgtt tgcaagcagc agattacgcg cagaaaaaaa 11650
ggatctcaag aagatccttt gatcttttct acggggtctg acgctcagtg 11700
gaacgaaaac tcacgttaag ggattttggt catgagatta tcaaaaagga 11750
tcttcaccta gatcctttta aattaaaaat gaagttttaa atcaatctaa 11800
agtatatatg agtaaacttg gtctgacagt taccaatgct taatcagtga 11850
ggcacctatc tcagcgatct gtctatttcg ttcatccata gttgcctgac 11900
tccccgtcgt gtagataact acgatacggg agggcttacc atctggcccc 11950
agtgctgcaa tgataccgcg agacccacgc tcaccggctc cagatttatc 12000
agcaataaac cagccagccg gaagggccga gcgcagaagt ggtcctgcaa 12050
ctttatccgc ctccatccag tctattaatt gttgccggga agctagagta 12100
agtagttcgc cagttaatag tttgcgcaac gttgttgcca ttgctacagg 12150
catcgtggtg tcacgctcgt cgtttggtat ggcttcattc agctccggtt 12200
cccaacgatc aaggcgagtt acatgatccc ccatgttgtg caaaaaagcg 12250
gttagctcct tcggtcctcc gatcgttgtc agaagtaagt tggccgcagt 12300
gttatcactc atggttatgg cagcactgca taattctctt actgtcatgc 12350
catccgtaag atgcttttct gtgactggtg agtactcaac caagtcattc 12400
tgagaatagt gtatgcggcg accgagttgc tcttgcccgg cgtcaatacg 12450
ggataatacc gcgccacata gcagaacttt aaaagtgctc atcattggaa 12500
aacgttcttc ggggcgaaaa ctctcaagga tcttaccgct gttgagatcc 12550
agttcgatgt aacccactcg tgcacccaac tgatcttcag catcttttac 12600
tttcaccagc gtttctgggt gagcaaaaac aggaaggcaa aatgccgcaa 12650
aaaagggaat aagggcgaca cggaaatgtt gaatactcat actcttcctt 12700
tttcaatatt attgaagcat ttatcagggt tattgtctca tgagcggata 12750
catatttgaa tgtatttaga aaaataaaca aataggggtt ccgcgcacat 12800
ttccccgaaa agtgccac 12818